# EUROPEAN PATENT APPLICATION

(11) **EP 4 847 497 A1**
(43) Date of publication of application: **02.12.2026**
(21) Application number: 25745358.9
(22) Date of filing: 23.01.2025
(51) Int. Cl.: C07D 401/12, A61K 49/00, A61K 51/04, A61K 31/4439, A61P 43/00, A61K 47/54, A61K 47/69

(54) **FIBROBLAST ACTIVATION PROTEIN-TARGETING MATERIAL BASED ON 3-STYRYLPYRIDINE STRUCTURE**

(30) Priority: 24.01.2024 KR 20240011228; 10.12.2024 KR 20240183001
(71) Applicant: Seoul National University R&DB Foundation, Seoul 08826 (KR); Seoul National University Hospital, Seoul 03080 (KR)
(72) Inventor: IM, Hyung-Jun, Suwon-si Gyeonggi-do 16229 (KR); LEE, Byung Chul, Hanam-si Gyeonggi-do 13014 (KR); KIM, Da Bin, Cheonan-si Chungcheongnam-do 31113 (KR); HWANG, Na Yeong, Icheon-si Gyeonggi-do 17358 (KR); CHOI, Ji Young, Seoul 05117 (KR)
(74) Representative: Müller, Christian Stefan Gerd
(86) International application number: PCT/KR2025/001333
(87) International publication number: WO 2025/159534

(57) **Abstract**

The present invention relates to a ligand compound that is activated by or binds to a fibroblast activation protein (FAP).

## Description

### Technical Field

The present invention relates to a ligand compound capable of specifically binding to fibroblast activation protein (FAP) and various uses thereof.

### Background Art

Fibroblast activation protein (FAP) is a transmembrane protein, a member of the serine protease family, and is characterized in that it is mainly expressed in activated fibroblasts and is rarely expressed in normal adult tissues. On the other hand, FAP is well known to be selectively overexpressed in various pathological conditions. In particular, FAP is highly expressed in 90% or more of reactive stromal fibroblasts of various epithelial malignancies (primary and metastatic), including epithelial malignancies (primary and metastatic), including lung, colorectal, bladder, ovarian and breast carcinomas, and is also strongly expressed in malignant mesenchymal tumors such as bone and soft tissue sarcomas. These specific expression patterns suggest that FAP plays an important role in the tumor microenvironment and support the possibility that FAP acts as an important mediator in the interaction between cancer cells and the stroma.

In addition, FAP has been found to play an important role in tissue remodeling and fibrosis processes in addition to tumor tissue. For example, high expression of FAP by activated fibroblasts in scar tissue has been observed, and recent studies have reported that FAP expression significantly increases in chronic fibrotic lesions in organs such as the liver, lungs, and colon. This suggests that FAP can be a potential therapeutic target not only for neoplastic diseases but also for various fibrosis-related diseases.

These characteristics of FAP offer great value as a target protein for the development of diagnostic and therapeutic agents. Accordingly, various binding ligands, prodrugs, and therapeutic agents have been developed to target FAP. These ligands have been designed to be selectively activated in lesion tissues where FAP is expressed or to bind to FAP, but there are several important limitations in existing technologies. First, there is a problem in that, since the active site of FAP shares high sequence and structural similarity with dipeptidyl peptidase-4 (DPP4), selective specificity for FAP is not sufficiently ensured. Second, there is significant overlap in substrate specificity between FAP and prolyl endopeptidase (PREP), so there is a possibility that a drug may be activated not only by FAP but also in various non-target tissues where DPP4 and PREP are expressed. This non-specific activation not only reduces the therapeutic effect of the drug but also increases the risk of causing unwanted adverse effects.

For example, DPP4 is known as a major enzyme that is expressed in various tissues of the human body, such as the pancreas, liver, and lungs, and is involved in blood glucose regulation. Therefore, if a FAP-targeting ligand is activated even by DPP4, there is a possibility that the ligand may cause adverse effects on metabolic processes, such as blood glucose regulation, due to the drug's off-target effects. Similarly, PREP is expressed in various tissues, such as the nervous and endocrine systems, and is involved in the degradation of specific peptide hormones. For this reason, if an FAP-targeting ligand exhibits pharmacological activity in unwanted tissues through non-specific interactions with PREP, the likelihood of adverse effects increases.

Therefore, there is a need for a novel approach that overcomes the limitations of existing FAP-targeting ligands, has higher specificity for FAP, and can prevent activation in non-target tissues. In particular, designing ligands capable of effectively targeting high expression of FAP in cancer or fibrotic disease lesions is a very important challenge. The present invention provides a novel ligand having high specificity for FAP, thereby overcoming the limitations of existing technologies and presenting the possibility of significantly improving the efficiency of diagnosis and treatment of FAP-related disease.

### DISCLOSURE

### Technical Problem

One object of the present invention is to provide a compound having a novel structure capable of targeting fibroblast activation protein (FAP).

Another object of the present invention is to provide a drug complex containing a novel structure provided in the present invention.

Still another object of the present invention is to provide the use of compounds provided in the present invention for the diagnosis or treatment of FAP-related disease.

However, the technical problems to be solved by the present invention are not limited to the problems mentioned above, and other problems not mentioned may be clearly understood by those skilled in the art from the following description.

### Technical Solution

Hereinafter, various embodiments described herein will be described with reference to figures. In the following description, numerous specific details are set forth, such as specific configurations, compositions, and processes, etc., in order to provide a thorough understanding of the present invention. However, certain embodiments may be practiced without one or more of these specific details, or in combination with other known methods and configurations. In other instances, known processes and preparation techniques have not been described in particular detail in order to not unnecessarily obscure the present invention. Reference throughout this specification to "one embodiment" or "an embodiment" means that a particular feature, configuration, composition, or characteristic described in connection with the embodiment is included in at least one embodiment of the present invention. Thus, the appearances of the phrase "in one embodiment" or "an embodiment" in various places throughout this specification are not necessarily referring to the same embodiment of the present invention. Additionally, the particular features, configurations, compositions, or characteristics may be combined in any suitable manner in one or more embodiments.

Fibroblast activation protein (FAP) is a protease belonging to the serine protease family, specifically the dipeptidyl peptidase IV (DPPIV) family. FAP protein is mainly expressed in activated fibroblasts and is highly expressed in the tumor microenvironment (TME) surrounding cancer cells, fibrotic tissue, wounds, or inflammatory sites. Therefore, the development of ligands for FAP as a target for diagnosing or treating these diseases is actively underway. Most of the FAP-targeting ligands developed to date include a quinoline derivative structure as an important moiety of their pharmacological core structure. However, the inventors of the present invention have made extensive efforts, and as a result, have found that, when a 4-hydroxystyrylpyridine structure rather than a quinoline is bonded to a glycine-proline derivative, the binding affinity of the derivative for FAP and the ability to inhibit FAP are significantly increased. In this case, the compatibility with the S1 pocket of FAP or the surrounding active site may vary depending on the bonding position in the pyridine structure. In the present invention, both the binding affinity for FAP and the effect of inhibiting FAP activity are improved by allowing hydroxystyryl and glycine-proline derivatives to be bonded at positions 5 and 2 of pyridine.

### FAP-Targeting Compound

According to one embodiment of the present invention, the present invention relates to a compound selected from the group consisting of a compound represented by Formula 1 below, a pharmaceutically acceptable salt thereof, an optical isomer thereof, a solvate thereof, and a hydrate thereof:

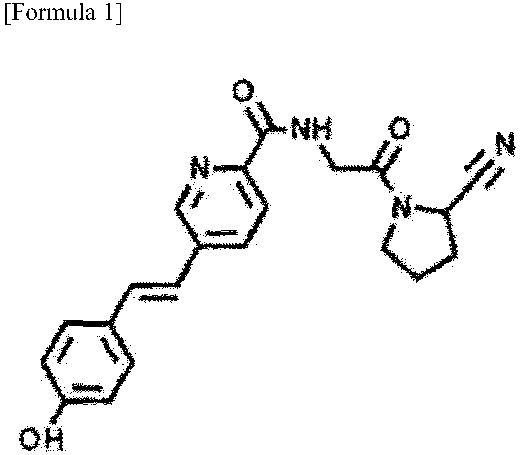

As described above, the compound represented by Formula 1 according to the present invention may specifically bind to fibroblast activation protein (FAP) and furthermore, inhibit the enzymatic activity of FAP. The compound provided in the present invention is a FAP inhibitor that may be used to prevent, alleviate, or treat FAP-related disease.

In addition, various substances such as drugs or fluorescent dyes may be linked to the above compound and used to diagnose or treat FAP-related disease.

According to another embodiment of the present invention, the present invention relates to a compound selected from the group consisting of a compound represented by Formula 2 below, a pharmaceutically acceptable salt thereof, an optical isomer thereof, a solvate thereof, and a hydrate thereof:

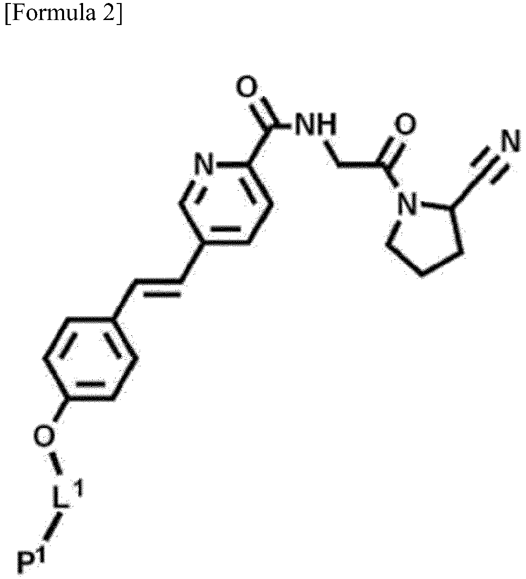

In Formula 2 above,
L¹ is a direct bond or a linker; and
P¹ is a radioactive moiety, a chelating agent, a fluorescent moiety, a photoacoustic reporting molecule, a Raman-active reporter molecule, a contrast agent, a detectable nanoparticle, an enzyme, an antifibrotic agent, an anti-inflammatory agent, an immunosuppressive agent, or a cytotoxic agent.

The linker is a group that links the FAP ligand to the moiety P¹ such as a drug, and examples thereof include both a releasable linker and a non-releasable linker. The linker may contain an atom selected from C, N, O, S, Si and P; C, N, O, S and P; or C, N,⃝ and S. In addition, the linker may be, but is not limited to, one or more selected from the group consisting of alkylene, cycloalkylene, arylalkylene, heteroarylalkylene, heteroalkylene, heterocycloalkylene, alkenylene, cycloalkenylene, arylalkenylene, heteroarylalkenylene, heteroalkenylene, heterocycloalkenylene, alkynylene, heteroalkynylene, arylene, heteroarylene, aminoacyl, oxyalkylene, aminoalkylene, diacid ester, dialkylsiloxane, amide, thioamide, thioether, thioester, ester, carbamate, hydrazone, thiazolidine, methylene alkoxy carbamate, disulfide, vinylene, imine, imidamide, phosphoramide, saccharides, phosphate ester, phosphoramide, carbamate, dipeptide, tripeptide, tetrapeptide, urea, guanidine, thiourea, disulfide, oxime, hydrazine, hydrazide, hydrazone, diaza bonds, triazole, triazolin, tetrazine, platinum complexes, and amino acids.

In the present invention, the radioactive moiety comprises a fluorescent drug, a radioisotope, a radioactive drug, or a combination thereof, and preferably may be selected from the group consisting of an alpha radiation-emitting isotope, a beta radiation-emitting isotope, a gamma radiation-emitting isotope, an Auger electron-emitting isotope, an X-ray-emitting isotope, and a fluorescence-emitting drug.

In the present invention, the radioisotope may comprise a radioactive metallic or non-metallic isotope which enables imaging or radiotherapy, and specific examples of the radioactive metallic or non-metallic isotope include, but are not limited to, ¹⁸F, ¹⁹F, ⁴³K, ⁴⁷Sc, ⁵¹Cr , ⁵⁷Co, ⁵⁸Co, ⁵⁹Fe, ⁶⁴Cu, ⁶⁷Cu, ⁶⁷Ga, ⁶⁸Ga, ⁷¹Ge, ⁷²As, ⁷²Se, ⁷⁵Br, ⁷⁶Br, ⁷⁷As, ⁷⁷Br, ⁸¹Rb, ⁸⁸Y, ⁹⁰Y, ⁹⁷Ru, ^{99m}Tc, ⁹⁹Mo, ¹⁰⁰Pd, ^{101m}Rh, ¹⁰³Pb, ¹⁰⁵Rh, ¹⁰⁹Pd, ¹¹¹Ag, ¹¹¹In, ¹¹³In, ¹¹⁹Sb, ¹²¹Sn, ¹²³I, ¹²⁴I, ¹²⁵I, ¹²⁷Cs, ¹²⁸Ba, ¹²⁹Cs, ¹³¹Cs, ¹³¹I, ¹³⁹La, ¹⁴⁰La, ¹⁴²Pr, ¹⁴³Pr, ¹⁴⁹Pm, ¹⁵¹Eu, ¹⁵³Eu, ¹⁵³Sm, ¹⁵⁹Gr, ¹⁶¹Tb, ¹⁶⁵Dy, ¹⁶⁶Ho, ¹⁶⁹Eu, ¹⁷⁵Yb, ¹⁷⁷Lu, ¹⁸⁶Re, ¹⁸⁸Re, ¹⁸⁹Re, ¹⁹¹Os, ¹⁹³Pt, ¹⁹⁴Ir, ¹⁹⁷Hg, ¹⁹⁸Au, ¹⁹⁹Ag, ¹⁹⁹Au, ²⁰¹Tl, ²⁰³Pb, ²¹¹At, ²¹²Bi, ²¹²Pb, ²¹³Bi, ²²⁵Ac, ²²⁷Th, ⁴⁴Sc, ⁴⁷Sc, ⁷⁷As, ¹¹⁰In, ¹¹¹In, ¹¹³In, ¹⁴⁹Tb, ¹⁵²Tb, ⁸⁶Y, ⁸⁸Y, ⁸³Sr, ⁸⁹Sr, ⁸⁹Zr, and ¹⁶⁶Dy.

In the present invention, the radioisotope may be a radioisotope for diagnostic imaging. In this case, examples of the radioisotope include, but are not limited to, ¹⁸F, ¹²³I, ¹²⁴I, ¹²⁵I, ⁶⁸Ga, ⁸⁹Zr, and ^{99m}Tc.

In the present invention, the radioisotope may be a therapeutic radioisotope. In this case, examples of the therapeutic radioisotope include, but are not limited to, ²²⁵Ac, ¹⁷⁷Lu, ⁶⁷Cu, ¹³¹I, ³²P, ⁹⁰Sr, ⁹⁰Y, ⁹⁹Mo, ¹⁸⁶Re, and ¹¹¹In, which are suitable for cancer treatment.

In the present invention, the radioisotope may be a transition metal. In this case, examples of the transition metal include, but are not limited to, ⁴⁴Sc, ⁴⁷Sc, ⁵¹Cr, ⁵¹Mn, ⁵²Mn, ⁵⁷Co, ⁵⁸Co, ⁵⁹Fe, ⁶⁴Cu, ⁶⁷Cu, ⁸⁶Y, ⁸⁸Y, ⁸⁹Zr, ⁹⁰Y, ⁹⁷Ru, ^{99m}Tc, ¹⁰⁰Pd, ^{101m}Rh, ¹⁰³Pd, ¹⁰⁵Rh, ¹⁰⁹Pd, ¹¹¹Ag, ¹⁷⁷Lu, ¹⁸⁶Re, ¹⁸⁸Re, ¹⁸⁹Re, ¹⁹¹Os, ¹⁹³Pt, ¹⁹⁴Ir, ¹⁹⁷Hg, ¹⁹⁸Au, ¹⁹⁹Ag, ¹⁹⁹Au, ²²⁵Ac, ²²⁶Th, and ²²⁷Th.

In the present invention, the radioisotope may be an s-block metal. In this case, examples of s-block metal include, but are not limited to, ⁴³K, ⁸¹Rb, ⁸³Sr, ⁸⁹Sr, ¹²⁷Cs, ¹²⁸Ba, ¹²⁹Cs, and ¹³¹Cs.

In the present invention, the radioisotope may be selected from among elements of groups 13 to 16 of the periodic table. In this case, examples of the radioisotope include, but are not limited to, ⁶⁷Ga, ⁶⁸Ga, ⁷¹Ge, ⁷²As, ⁷²Se, ⁷⁷As, ¹¹⁰In, ¹¹¹In, ¹¹³In, ¹¹⁹Sb, ¹²¹Sn, ²⁰¹Tl, ²⁰³Pb, ²¹²Bi, ²¹²Pb, and ²¹³Bi.

In the present invention, the radioisotope may be a halogen. In this case, examples of the halogen include, but are not limited to, ¹⁸F, ¹⁹F, ⁷⁵Br, ⁷⁶Br, ⁷⁷Br, ¹²³I, ¹²⁴I, ¹²⁵I, ¹³¹I, and ²¹¹At.

In the present invention, the radioisotope may be a lanthanide. In this case, examples of the lanthanide include, but are not limited to, ¹³⁹La, ¹⁴⁰La, ¹⁴²Pr, ¹⁴³Pr, ¹⁴⁹Pm, ¹⁵¹Eu, ¹⁵³Eu, ¹⁵³Sm, ¹⁵⁹Gr, ¹⁴⁹Tb, ¹⁵²Tb, ¹⁶¹Tb, ¹⁶⁵Dy, ¹⁶⁶Dy, ¹⁶⁶Ho, ¹⁶⁹Eu, and ¹⁷⁵Yb.

In the present invention, the radioisotope may be an actinide. In this case, examples of the actinide include, but are not limited to, ²²⁵Ac, ²²⁶Th, and ²²⁷Th.

In the present invention, the radioisotope may comprise a combination of at least two radioisotopes. In this case, examples of the combination include, but are not limited to, combinations of ⁶⁸Ga and ¹⁷⁷Lu; ¹⁸F and ¹⁷⁷Lu; ¹¹¹In and ¹⁷⁷Lu; ⁶⁸Ga and ⁹⁰Y; ¹⁸F and ⁹⁰Y; ¹¹¹In and ⁹⁰Y; ⁶⁸Ga and ²²⁵Ac; ¹⁸F and ²²⁵Ac; and ¹¹¹In and ²²⁵Ac.

In the present invention, the radioactive moiety may further comprise at least one non-radioactive or non-toxic carrier metal. In this case, the carrier metal may comprise a carrier metal for MRI imaging, for example, Fe, or a carrier metal for X-ray contrast imaging, for example, Bi, without being limited thereto.

In the present invention, the chelating agent may be a chelator for a non-radioactive isotope, a chelator for a radiometal or paramagnetic ion, including a radioactive isotope. Here, the chelating agent may comprise any chelator known in the art, see, e.g., Parus et al., "Chemistry and bifunctional chelating agents for binding (177)Lu," Curr Radiopharm. 2015; 8(2):86-94; Wangler et al., "Chelating agents and their use in radiopharmaceutical sciences," Mini Rev Med Chem. 2011 October; 11(11):968-83; Liu, "Bifunctional Coupling Agents for Radiolabeling of Biomolecules and Target-Specific Delivery of Metallic Radionuclides," Adv Drug Deliv Rev. 2008 September; 60(12): 1347-1370.

In the present invention, examples of the chelating agent include, but are not limited to, 1,4,7,10-tetraazacyclododecane-N,N',N,N'-tetraacetic acid (DOTA), ethylenediaminetetraacetic acid (EDTA), 1,4,7-triazacyclononane-1,4,7-triacetic acid (NOTA), triethylenetetramine (TETA), iminodiacetic acid (IDA), diethylenetriamine-N,N,N',N',N"-pentaacetic acid (DTPA), and 6-hydrazinopyridine-3-carboxylic acid (HYNIC).

In the present invention, the chelating agent may comprise a radioisotope or paramagnetic ion chelated with a chelator, and may further comprise a non-radioactive isotope. The types of radioisotopes overlap with those described above, and thus detailed description thereof will be omitted below. Meanwhile, examples of the paramagnetic ion include, but are not limited to, chromium(III), manganese(II), iron(III), iron(II), cobalt(II), nickel(II), copper(II), neodymium(III), samarium(III), ytterbium(III), gadolinium(III), vanadium(II), terbium(III), dysprosium(III), holmium(III), erbium(III), or combinations of these paramagnetic ions.

In the present invention, the fluorescent moiety acts as a detectable label and may be selected from, for example, a fluorescent dye, a fluorescent protein, a fluorescent peptide, a fluorescent material, or combinations thereof.

In the present invention, examples of the fluorescent dye include, but are not limited to, xanthens, acridines, oxazines, cyanines, styryl dyes, coumarines (such as Coumarin 343, methoxycoumarin and dialkylaminocoumarin), porphines, metal-ligand-complexes, fluorescent proteins, nanocrystals, perylenes, boron-dipyrromethenes and phtalocyanines, cyanine, fluorescein and fluorescein derivatives, rhodamine and rhodamine derivatives, Alexa Fluor, Dylight Fluor (such as DyLight547 and Dylight647), Hylight Fluor (such as HiLyte Fluor 647, HiLyte Fluor 680 and HiLyte Fluor 750), IRDyes (such as IR Dye 800, IRDye 800CW, IRDye 800RS and IRDye 700DX), Dy fluros (such as Dy677, Dy676, Dy682, Dy752 and Dy780), VivoTag Fluor (such as VivoTag-680, VivoTag-S680 and VivoTag-S750), ATTO dyes, BODIPY Fluor (such as BODIPY FL, BODIPY R6G, BODIPY TMR, BODIPY TR, BODIPY 530/550, BODIPY 558/568, BODIPY 564/570, BODIPY 576/589, BODIPY 581/591, BODIPY 630/650 and BODIPY 650/665), carbocyanine, indocarbocyanine, oxacarbocyanine, thuicarbocyanine, merocyanine, polymethine, a boron-dipyrromethane (BODIPY) dye, ADS780WS, ADS830WS, and ADS832WS, and other flurophores that will be known to those skilled in the art.

To further exemplify, the fluorescent moiety in the present invention may be selected from the group consisting of Cy3, Cy5, Cy5.5 (also known as Cy5++), Cy2, CY7, CY7.5, fluorescein isothiocyanate (FITC), 4',5'-dichloro-2',7'-dimethoxy-fluorescein, naphthofluorescein, 2',4',5',7'-tetra-bromosulfone-fluorescein, tetramethylrhodamine isothiocyanate (TRITC), phycoerythrin, Cy7, fluorescein (FAM), Cy3, Cy3.5 (also known as Cy3++), Texas Red, Texas Red-X, Marina Blue, Oregon Green 488, Oregon Green 500, Oregon Green 514, Pacific Blue, PyMPO, AMCA, AMCA-S, Cascade Blue, Cascade Yellow, DM-NERF, Eosin, Erythrosin, FAM, LightCycler fluors (such as LightCycler-Red 640 and LightCycler Red 705), tetramethylrhodamine (TMR), rhodamine, rhodamine derivative (ROX), hexachlorofluorescein (HEX), rhodamine 6G (R6G), carboxy-X-rhodamine, Lissamine rhodamine B, Pyrene, Rhodamine B, Rhodamine 6G, Rhodamine Green, Rhodol Red, Rhodol Green, tetramethyl-rhodamine, carboxytetramethylrhodamine, the rhodamine derivative JA133, Alexa fluorescent dyes (such as Alexa Fluor 350, Alexa Fluor 488, Alexa Fluor 546, Alexa Fluor 633, Alexa Fluor 555, Alexa Fluor 647, AlexaFluor 660, AlexaFluor 680, AlexaFluor 700, AlexaFluor 750 and AlexaFluor 790), 4',6-diamidino-2-phenylindole (DAPI), propidium iodide, AMCA, Spectrum Green, Spectrum Orange, Spectrum Aqua, Lissamine, and fluorescent transition metal complexes, such as europium. Fluorescent compounds that may be used also include fluorescent proteins, such as GFP (green fluorescent protein), enhanced GFP (EGFP), blue fluorescent protein and derivatives (BFP, EBFP, EBFP2, Azurite, mKalama1), cyan fluorescent protein and derivatives (CFP, ECFP, Cerulean, CyPet) and yellow fluorescent protein and derivatives (YFP, Citrine, Venus, YPet). See also WO 2008/142571, WO 2009/056282, and WO 99/22026 (all of which are incorporated by reference).

In addition, in the present invention, examples of the detectable moiety include biological fluorophores (such as fluorescent polypeptide or peptide), including, but not limited to, green fluorescent protein (GFP) derivatives of GFP (e.g., EBFP, EBFP2, Azurite, mKalama1, ECFP, Cerulean, CyPet, YFP, Citrine, Venus, Ypet) and R-Phycoerythrin.

In addition, in the present invention, examples of the photoacoustic reporting molecule include, but are not limited to, indocyanine-green (ICG), Alexa Fluor 750, Evans Blue, BHQ3, QXL680, IRDye880CW, MMPSense 680, Methylene Blue, PPCy-C8, and Cypate-C 18.

In the present invention, the detectable moiety may comprise a detectable nanoparticle selected from the group consisting of a plasmonic nanoparticle, a quantum dot, a nanodiamond, a polypyrrole nanoparticle, a copper sulfide nanoparticle, a graphene nanosheet, an iron oxide-gold core-shell nanoparticle, a Gd₂O₃ nanoparticle, a single-walled carbon nanotube, a dye-loaded perfluorocarbon nanoparticle, and a superparamagnetic iron oxide nanoparticle.

In addition, in the present invention, the detectable moiety may comprise a quantum dot, for example, an infrared-emitting quantum dot, without being limited thereto.

In the present invention, the detectable moiety may comprise a Raman-active reporter molecule, such as a single-walled carbon nanotube (SWNT) or a surface-enhanced Raman scattering (SERS) agent. Here, examples of surface-enhanced Raman scattering agent may be metal nanoparticles labeled with a Raman-active reporter molecule. In this case, examples of fluorescent dyes that may also be used as Raman-active reporter molecules include, but are not limited to, Cy3, Cy5, rhodamine, and chalcogenopyrylium dyes.

In the present invention, examples of the enzyme include, but are not limited to, horseradish peroxidase (HRP), alkaline phosphatase (AP), glucose oxidase, and β-galactosidase.

In the present invention, examples of the antifibrotic agent include, but are not limited to, pirfenidone or receptor tyrosine kinase inhibitors (RTKIs) such as Nintedanib, Sorafenib and other RTKIs, or angiotensin II (AT1) receptor blockers, or CTGF inhibitor, or any antifibrotic compound susceptible to interfere with the TGFβ and BMP-activated pathways including activators of the latent TGFβ complex such as MMP2, MMP9, THBS1 or cell-surface integrins, TGFβ receptors type I (TGFBRI) or type II (TGFBRII) and their ligands such as TGFβ, Activin, inhibin, Nodal, anti-Müllerian hormone, GDFs or BMPs, auxiliary co-receptors (also known as type III receptors), or components of the SMAD-dependent canonical pathway including regulatory or inhibitory SMAD proteins, or members of the SMAD-independent or non-canonical pathways including various branches of MAPK signaling, TAK1, Rho-like GTPase signaling pathways, phosphatidylinositol-3 kinase/AKT pathways, TGFβ-induced EMT process, or canonical and non-canonical Hedgehog signaling pathways including Hh ligands or target genes, or any members of the WNT, or Notch pathways which are susceptible to influence TGFβ.

In the present invention, examples of the anti-inflammatory agent and/or immunosuppressive agent include, but is not limited to, glucocorticoids, NSAIDs, cyclophosphamide, nitrosourea, folic acid analogs, purine analogs, pyrimidine analogs, methotrexate, azathioprine, mercaptopurine, cyclosporine, myriocin, tacrolimus, sirolimus, mycophenolic acid derivatives, fingolimod and other sphingosine-1-phosphate receptor modulators, and monoclonal and/or polyclonal antibodies against targets such as proinflammatory cytokines and proinflammatory cytokine receptors, T-cell receptor and integrins.

In the present invention, the cytotoxic agent is understood as a "chemotherapeutic agent" or an "anti-neoplastic agent", and includes chemical agents that prevent the development, maturation, or spread of neoplastic cells on the tumor cell. In the present invention, the types of cytotoxic agents are not particularly limited and may include natural or synthetic chemical compounds, or biological molecules, such as proteins, polypeptides, etc., and may also include large numbers of antineoplastic agents available in commercial use, in clinical evaluation and in pre-clinical development. Examples of the cytotoxic agent include alkylating agents, for example, nitrogen mustards, ethyleneimine compounds, alkyl sulphonates and other compounds with an alkylating action such as nitrosoureas, cisplatin and dacarbazine; antimetabolites, for example, folic acid, purine or pyrimidine antagonists; mitotic inhibitors, for example, vinca alkaloids and derivatives of podophyllotoxin; cytotoxic antibiotics and camptothecin derivatives. Specific examples of the cytotoxic agent include, but are not limited to, amifostine (ethyol), cabazitaxel, cisplatin, dacarbazine (DTIC), dactinomycin, docetaxel, mechlorethamine, streptozocin, cyclophosphamide, carrnustine (BCNU), lomustine (CCNU), doxorubicin (adriamycin), doxorubicin lipo (doxil), gemcitabine (gemzar), daunorubicin, daunorubicin lipo (daunoxome), procarbazine, ketokonazole, mitomycin, cytarabine, etoposide, methotrexate, 5-fluorouracil (5-FU), vinblastine, vincristine, bleomycin, paclitaxel (taxol), docetaxel (taxotere), aldesleukin, asparaginase, busulfan, carboplatin, cladribine, camptothecin, CPT-11, 10-hydroxy-7-ethyl-camptothecin (SN38), dacarbazine, floxuridine, fludarabine, hydroxyurea, ifosfamide, idarubicin, mesna, interferon alpha, interferon beta, irinotecan, mitoxantrone, topotecan, leuprolide, megestrol, melphalan, mercaptopurine, plicamycin, mitotane, pegaspargase, pentostatin, pipobroman, plicamycin, streptozocin, tamoxifen, teniposide, testolactone, thioguanine, thiotepa, uracil mustard, vinorelbine, chlorambucil, and combinations thereof.

In one embodiment of the present invention, the compound represented by Formula 2 may be a compound represented by Formula 3 below, without being limited thereto:

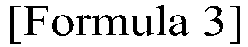

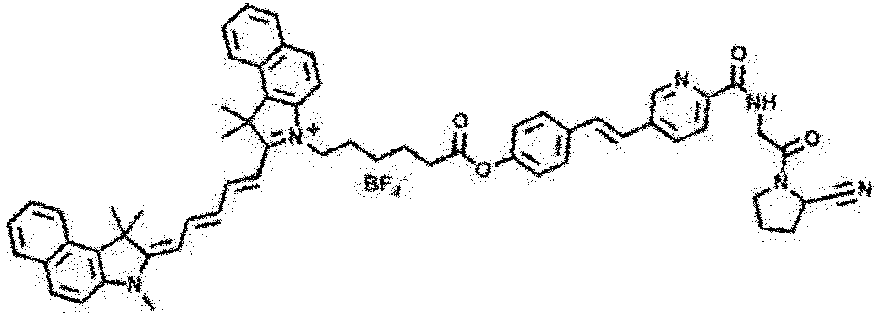

The present invention also provides pharmaceutically acceptable salts of the above-described compounds. The pharmaceutically acceptable salts are salts which are usually considered by those skilled in the art to be suitable for medical applications, e.g., because they are not harmful to subjects which may be treated with the salts, or which give rise to side effects which are tolerable within the respective treatment. Usually, the pharmaceutically acceptable salts are salts which are considered as acceptable by the regulatory authorities, such as the US Food and Drug Administration (FDA), the European Medicines Agency (EMA), or the Japanese Ministry of Health, Labor and Welfare Pharmaceuticals and Medical Devices Agency (PMDA). However, the present invention in principle also encompasses salts of the compounds according to the present invention which are as such not pharmaceutically acceptable, e.g., as intermediates in the production of the compounds according to the present invention or physiologically functional derivatives thereof, or as intermediates in the production of pharmaceutically acceptable salts of the compounds according to the present invention or physiologically functional derivatives thereof. Said salts include water-insoluble salts and, particularly, water-soluble salts.

In each case, those skilled in the art can readily determine whether a certain compound according to the present invention or a physiologically functional derivative thereof can form a salt, i.e., whether the compound according to the present invention or a physiologically functional derivative thereof has a group which may carry a charge, such as, for example, an amino group, a carboxylic acid group, etc.

Exemplary salts of the compounds of the present invention are acid addition salts or salts with bases, particularly pharmaceutically acceptable inorganic and organic acid addition salts and salts with bases customarily used in pharmacy, which are either water insoluble or, particularly, water-soluble acid addition salts. Salts with bases may, depending on the substituents of the compounds of the present invention, also be suitable. Acid addition salts may, for example, be formed by mixing a solution of a compound of the present invention with a solution of a pharmaceutically acceptable acid such as hydrochloric acid, sulfuric acid, fumaric acid, maleic acid, succinic acid, acetic acid, benzoic acid, citric acid, tartaric acid, carbonic acid, or phosphoric acid. Likewise, pharmaceutically acceptable base addition salts may include alkali metal salts (e.g., sodium or potassium salts); alkaline earth metal salts (e.g., calcium or magnesium salts); and salts formed with suitable organic ligands (e.g., ammonium, quaternary ammonium and amine cations formed using counteranions such as halide, hydroxide, carboxylate, sulfate, phosphate, nitrate, alkyl sulfonate and aryl sulfonate). Illustrative examples of pharmaceutically acceptable salts include, but are not limited to, acetate, adipate, alginate, arginate, ascorbate, aspartate, benzenesulfonate, benzoate, bicarbonate, bisulfate, bitartrate, borate, bromide, butyrate, calcium edetate, camphorate, camphorsulfonate, camsylate, carbonate, chloride, citrate, digluconate, dihydrochloride, dodecylsulfate, edetate, edisylate, ethanesulfonate, formate, fumarate, galactate, galacturonate, gluconate, glutamate, glycerophosphate, hemisulfate, heptanoate, hexanoate, hexylresorcinate, hydrobromide, hydrochloride, hydroiodide, 2-hydroxy-ethanesulfonate, hydroxynaphthoate, iodide, isobutyrate, isothionate, lactate, laurate, lauryl sulfate, malate, maleate, malonate, mandelate, methanesulfonate (mesylate), methylsulfate, 2-naphthalenesulfonate, nicotinate, nitrate, oleate, oxalate, palmitate, pantothenate, pectinate, persulfate, 3-phenylpropionate, phosphate/diphosphate, phthalate, picrate, pivalate, polygalacturonate, propionate, salicylate, stearate, sulfate, suberate, succinate, tannate, tartrate, tosylate, undecanoate, valerate, and the like.

Salts, which are not pharmaceutically acceptable and which can be obtained, for example, as process products during the production of the compounds according to the present invention on an industrial scale, are also encompassed by the present invention and, if desired, may be converted into pharmaceutically acceptable salts by processes known to those skilled in the art.

Meanwhile, the compounds according to the present invention may have an asymmetric carbon center, and thus may exist as R or S isomers or racemic compounds, and all of these optical isomers and mixtures may be included in the scope of the present invention.

In addition, the compounds of the present invention as well as salts thereof may contain various amounts of solvent when isolated, for example, in crystalline form,. Therefore, solvates and in particular hydrates of the compounds of the present invention, and also solvates and in particular hydrates of the salts of the compounds of the present invention may be included within the scope of the present invention. More particularly, the present invention may include hydrates of the compounds, salts, and/or physiologically functional derivatives according to the present invention, which comprise one, two or one half water molecule, with respect to their stoichiometry.

According to another embodiment of the present invention, the present invention relates to a liposome complex comprising a compound represented by Formula 4:

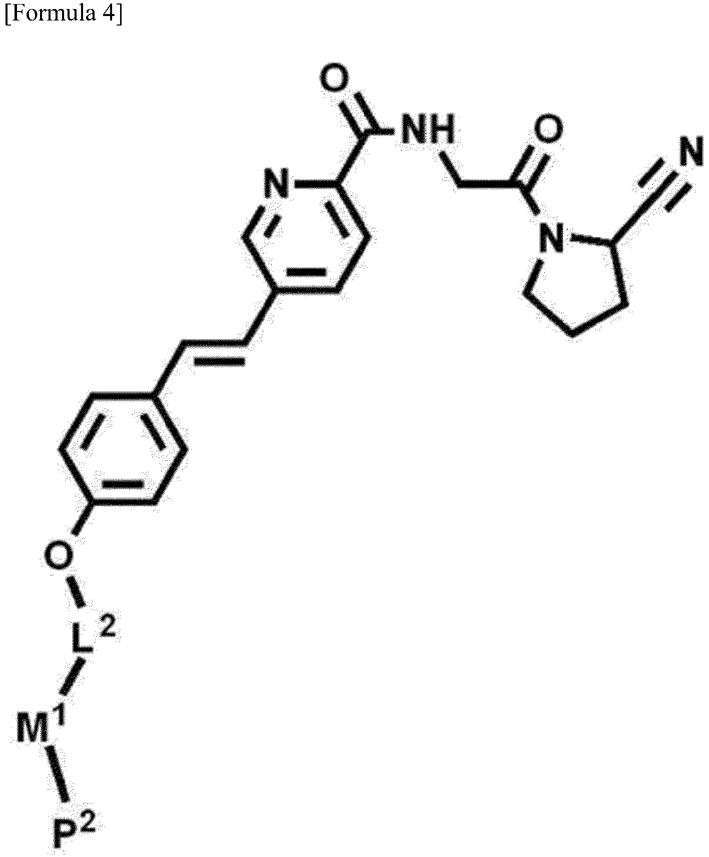

In Formula 4 above,
L² is a direct bond or a linker;
M¹ is one or more hydrophilic polymer radicals selected from the group consisting of polyethylene glycol (PEG), polycarboxybetaine (pCB), polysulfobetaine (pSB), phosphobetaine-based polymers, methoxypolyethylene glycol (mPEG), polyethylene glycol monomethyl ether, phosphatidylcholine (PC), phosphatidylethanolamine (PE), phosphatidylglycerol (PG), phosphatidylinositol (PI), phosphatidylserine (PS), monodisperse polyethylene glycol (monodisperse PEG), branched polyethylene glycol (branched PEG), and multi-arm polyethylene glycol (multi-arm PEG); and
P² is one or more phospholipid radicals selected from the group consisting of 1,2-dimyristoyl-sn-glycero-3-phosphocholine (DMPC), 1,2-dihexanoyl-sn-glycero-3-phosphocholine, 1,2-diheptanoyl-sn-glycero-3-phosphocholine, 1,2-dioctanoyl-sn-glycero-3-phosphocholine, 1,2-dinanoyl-sn-glycero-3-phosphocholine, 1,2-didecanoyl-sn-glycero-3-phosphocholine, 1,2-diundecanoyl-sn-glycero-3-phosphocholine, 1,2-dilauroyl-sn-glycero-3-phosphocholine (DLPC), 1,2-ditridecanoyl-sn-glycero-3-phosphocholine, 1,2-dipentadecanoyl-sn-glycero-3-phosphocholine, 1,2-dipalmitoyl-sn-glycero-3-phosphocholine (DPPC), 1,2-diheptadecanoyl-sn-glycero-3-phosphocholine, 1,2-distearoyl-sn-glycero-3-phosphocholine (DSPC), 1,2-distearoyl-sn-glycero-3-phosphoethanolamine (DSPE), 1,2-dinonadecanoyl-sn-glycero-3-phosphocholine, 1,2-diarachidoyl-sn-glycero-3-phosphocholine, 1,2-dihenarachidoyl-sn-glycero-3-phosphocholine, 1,2-dibehenoyl-sn-glycero-3-phosphocholine, 1,2-ditricosanoyl-sn-glycero-3-phosphocholine, 1,2-dilignoceroyl-sn-glycero-3-phosphocholine, hydrogenated phosphatidylcholine, 1,2-dioleoyl-sn-glycero-3-phosphocholine (DOPC), L-α-phosphatidylcholine (HSPC), 1-myristoyl-2-steroyl-sn-glycero-3-phosphocholine (MSPC), and 1-myristoyl-2-palmitoyl-sn-glycero-3-phosphocholine (MPPC).

In the present invention, L² may be, but is not limited to, a direct bond or a linker selected from the group consisting of -NH-(C=O)-, -NH-(C=S), -(C=O)-NH-, -(C=S)-NH-, -(C=O)-O-, -O-(C=O)-, -(C=O)-(C(R¹)(R²))r-, -(C=O)-(C(R¹)(R²))r-(C=O)-, -NH-(C=O)-NH-, -NH-(C=S)-NH-, -O-(C=O)-NH-, -NH-(C=O)-O-, -O-, -S-, and -S-S-. Here, r may be an integer ranging from 0 to 3; R¹ and R² may be each independently hydrogen or - (CH₂)s-C(R³)(R⁴); s may be an integer ranging from 0 to 4; R³ and R⁴ may be each independently selected from the group consisting of hydrogen, a hydroxyl group, a sulfhydryl group (-SH), an amine group (-NH₂), a C₁-C₆ alkyl group, a phenyl group, and a phenoxy group.

In the present invention, M¹ may be a radical form of polyethylene glycol (PEG), without being limited thereto.

In the present invention, P² may be a radical form of 1,2-distearoyl-sn-glycero-3-phosphoethanolamine (DSPE), without being limited thereto.

In the present invention, the liposome complex may be represented by Formula 5 below, without being limited thereto:

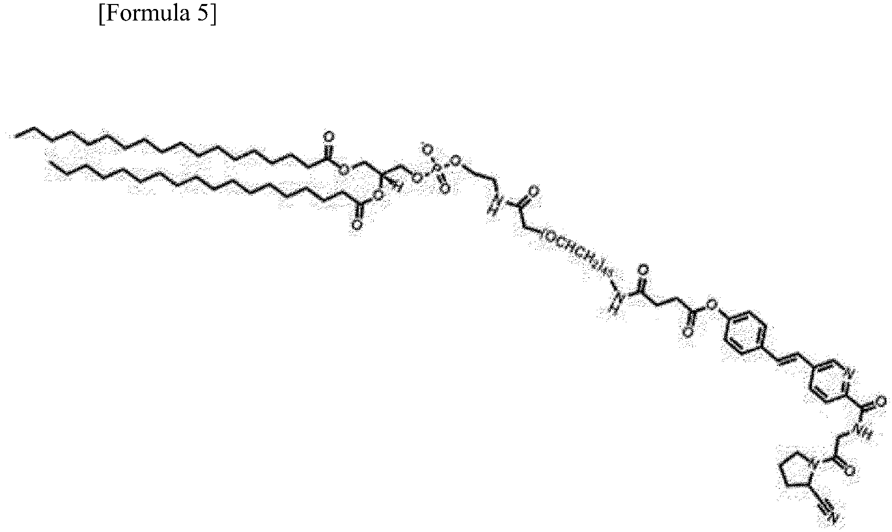

In the present invention, the size of the liposome complex may be, for example, 50 nm to 200 nm, without being limited thereto.

In the present invention, a radioactive moiety, a chelating agent, a fluorescent moiety, a photoacoustic reporting molecule, a Raman-active reporter molecule, a contrast agent, a detectable nanoparticle, an enzyme, an antifibrotic agent, an anti-inflammatory agent, an immunosuppressive agent, or a cytotoxic agent may be loaded into the liposome complex. Accordingly, the liposome complex of the present invention may be applied for the diagnosis or treatment of FAP-mediated disease. The description of a radioactive moiety, a chelating agent, a fluorescent moiety, etc., which may be contained within the liposome complex, overlaps with the above description, and thus detailed description thereof will be omitted below.

### Pharmaceutical Composition

According to another embodiment of the present invention, the present invention relates to a pharmaceutical composition comprising, as an active ingredient, a compound selected from the above-described compound, a pharmaceutically acceptable salt thereof, an optical isomer thereof, a hydrate thereof, and a solvate thereof.

The pharmaceutical composition provided in the present invention may be used for the prevention, treatment or diagnosis of FAP-mediated disease or disorder depending on the type of drug that is linked to a portion of the structure of the compound, which acts as a FAP ligand.

In the present invention, FAP-mediated disease may be a disease associated with proliferation, tissue remodeling, chronic inflammation, obesity, glucose intolerance, or insulin insensitivity.

In the present invention, the FAP-mediated disease may be a tumor or cancer, and more specifically, may be breast cancer, colorectal cancer, ovarian cancer, prostate cancer, pancreatic cancer, kidney cancer, lung cancer, melanoma, fibrosarcoma, osteosarcoma, connective tissue sarcoma, renal cell carcinoma, giant cell carcinoma, squamous cell carcinoma, leukemia, skin cancer, soft tissue cancer, liver cancer, gastrointestinal carcinoma, or adenocarcinoma, without being limited thereto.

In the present invention, the FAP-mediated disease may be, but is not limited to, fibrotic disease, wound healing, keloid formation, osteoarthritis, rheumatoid arthritis and related disorders involving cartilage degradation, atherosclerotic disease, Crohn's disease, or type II diabetes.

In the present invention, the "pharmaceutical composition" may be characterized in that it is in the form of a capsule, tablet, granule, injectable preparation, ointment, powder, or beverage, and the pharmaceutical composition may be characterized in that it is intended for animals, specifically humans.

For use, the pharmaceutical composition may be formulated in the form of oral preparations such as powders, granules, capsules, tablets, and aqueous suspensions, preparations for external use, suppositories, and sterile injectable solutions, according to the respective conventional methods, without being limited thereto. The pharmaceutical composition according to the present invention may contain pharmaceutically acceptable carriers. As the pharmaceutically acceptable carriers, a binder, a lubricant, a disintegrant, an excipient, a solubilizer, a dispersant, a stabilizer, a suspending agent, a colorant, a flavoring agent, and the like may be used for oral administration; a buffer, a preservative, a pain-relieving agent, a solubilizer, an isotonic agent, a stabilizer, and the like may be used for injection; and a base, an excipient, a lubricant, a preservative, and the like may be used for topical administration. The pharmaceutical composition of the present invention may be prepared in various dosage forms by being mixed with the pharmaceutically acceptable carriers as described above. For example, for oral administration, the pharmaceutical composition may be prepared in the form of tablets, troches, capsules, elixirs, suspensions, syrups, wafers, or the like. For injection, the pharmaceutical composition may be prepared in the form of unit dosage ampoules or in multiple-dosage forms. In addition, the pharmaceutical composition may be formulated into solutions, suspensions, tablets, capsules, sustained-release preparations, or the like.

Meanwhile, examples of carriers, excipients and diluents suitable for formulation include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, or mineral oil. In addition, the pharmaceutical composition of the present invention may further contain a filler, an anticoagulant, a lubricant, a wetting agent, a fragrance, an emulsifier, a preservative, or the like.

In the present invention, the pharmaceutical composition may further comprise a radical scavenger in addition to the compound of the present invention or a pharmaceutically acceptable salt thereof. The radical scavenger may be used to prevent radiolysis. Radiolysis is the process in which the ionization of oxygen or water molecules induced by the radionuclide leads to the formation of other reactive species, such as superoxide, hydrogen peroxide, hydrogen radicals, ozone and hydroxyl radicals. These reactive species can further cause damage to DNA and other cellular structures. In some embodiments, the radical scavenger is an antioxidant selected from carnosic acid, green tea extract, apigenin, diosmine, rosmarinic acid, lipoic acid, beta carotene, L-ascorbic acid (vitamin C), N-acetyl cysteine (NAC), δ-tocopherol, rutin, amifostine, resveratrol, gentisic acid, and gallic acid. In some embodiments, the radical scavenger may be an antioxidant selected from gallic acid, L-ascorbic acid and N-acetyl cysteine (NAC), without being limited thereto.

The routes of administration of the pharmaceutical composition include, but are not limited to, oral, intravenous, intramuscular, intraarterial, intramedullary, intradural, intracardiac, transdermal, subcutaneous, intraperitoneal, intranasal, enteral, topical, sublingual, or rectal administration. Oral or parenteral administration is preferred. The "parenteral" includes subcutaneous, transdermal, intravenous, intramuscular, intra-articular, intra-synovial, intrasternal, intradural, intra-lesional and intra-cranial injection or infusion techniques. The pharmaceutical composition of the present invention may also be formulated as suppositories for intrarectal administration.

In addition, the pharmaceutical composition may vary depending on a variety of factors, including the activity of a specific compound used, the patient's age, body weight, general health status, sex and diet, the time of administration, the route of administration, the rate of excretion, drug combination, and the severity of a specific disease to be prevented or treated. Although the administration of the pharmaceutical composition may vary depending on the patient's condition and body weight, the severity of disease, the drug form, and the route and duration of administration, it may be appropriately selected by those skilled in the art, and the pharmaceutical composition may be administered at a dose of 0.0001 to 50 mg/kg/day or 0.001 to 50 mg/kg/day. The administration may be done once a day or several times a day. The dose is not intended to limit the scope of the present invention in any way. The pharmaceutical composition according to the present invention may be formulated in the form of pills, sugar-coated tablets, capsules, liquids, gels, syrups, slurries, or suspensions.

The pharmaceutical composition of the present invention may be used alone or in combination with surgery, radiotherapy, hormone therapy, chemotherapy, and methods that use biological response modifiers.

### Use for FAP Imaging

According to one embodiment of the present invention, the present invention relates to an imaging composition for FAP detection comprising a compound provided in the present invention as an active ingredient.

According to another embodiment of the present invention, the present invention relates to a method for imaging FAP-expressing cells, organ or tissue, comprising: a step of administering the above-described pharmaceutical composition to a subject; and a step of imaging the tissue of the subject.

In the present invention, the "subject" refers to an animal (e.g., mammal or non-mammal). In one example, the subject may be a human or a primate other than a human. As an example, the subject may be a laboratory mammal (e.g., mouse, rat, rabbit, hamster, etc.). As an example, the subject may be an agricultural mammal (e.g., equine, ovine, bovine, porcine, camelid, etc.) or domestic mammal (e.g., canine, feline, etc.). Preferably, the subject may be a human.

When a pharmaceutical composition containing the compound according to the present invention is administered to a subject for the purpose of FAP imaging as described above, a drug (payload) that is linked to the FAP ligand of the compound may be a radioisotope for detection, or a chelating agent containing the same, a fluorescent moiety, a photoacoustic reporting molecule, a Raman-active reporter molecule, a contrast agent, a detectable nanoparticle, or an enzyme.

In the present invention, a radioisotope suitable for imaging as described above may be ⁴⁴Sc, ⁴⁷Sc, ⁵¹Cr, ⁵¹Mn, ⁵²Mn, ⁵⁷Co, ⁵⁸Co, ⁵⁹Fe, ⁶⁴Cu, ⁶⁷Cu, ⁸⁶Y, ⁸⁸Y, ⁸⁹Zr, ⁹⁰Y, ⁹⁷Ru, ^{99m}Tc, ⁹⁹Mo, ¹⁰⁰Pd, ^{101m}Rh, ¹⁰³Pd, ¹⁰⁵Rh, ¹⁰⁹Pd, ¹¹¹Ag, ¹⁷⁷Lu, ¹⁸⁶Re, ¹⁸⁸Re, ¹⁸⁹Re, ¹⁹¹Os, ¹⁹³Pt, ¹⁹⁴Ir, ¹⁹⁷Hg, ¹⁹⁸Au, ¹⁹⁹Ag, ¹⁹⁹Au, ²²⁵Ac, ²²⁶Th or ²²⁷Th, and in particular may be ⁴⁴Sc, ⁴⁷Sc, ⁶⁴Cu, ⁸⁹Zr, ⁹⁰Y, ^{99m}Tc, ¹⁷⁷Lu, ¹⁸⁶Re, ¹⁸⁸Re, ²²⁵Ac, ²²⁶Th or ²²⁷Th, without being limited thereto.

In the present invention, the FAP-expressing cells, organ or tissue may include prostate tissue, kidney tissue, brain tissue, vascular tissue, fibrotic tissue, tumor tissue, or cancer tissue.

In the present invention, the fibrotic tissue may be a liver, lung, heart, vascular system, articular or interstitial tissue, pancreas, skin, mouth, digestive tract, brain, breast, bone marrow, peritoneum, or kidney tissue in which a fibrotic condition has occurred, without being limited thereto.

In the present invention, the tumor or cancer tissue may be a tissue derived from breast cancer, colorectal cancer, ovarian cancer, prostate cancer, pancreatic cancer, kidney cancer, lung cancer, melanoma, fibrosarcoma, osteosarcoma, connective tissue sarcoma, renal cell carcinoma, giant cell carcinoma, squamous cell carcinoma, leukemia, skin cancer, soft tissue cancer, liver cancer, gastrointestinal carcinoma or adenocarcinoma, without being limited thereto.

In the present invention, the technique for imaging may be, but is not limited to, single photon emission computed tomography (SPECT), positron emission tomography (PET), or a combination of positron emission tomography and computed tomography (PET-CT).

In the present invention, the imaging composition may further comprise a second compound or a composition containing the same. In addition, the imaging method according to the present invention may further comprise a step of administering the second compound or a composition containing the same to a subject. In this case, the second compound or the composition containing the same may not target FAP.

As an example, the second compound or the composition containing the second compound may be a second imaging agent. For example, the second imaging agent may be an MRI imaging agent or a CT imaging agent, and specific examples thereof include, but are not limited to, gadoteridol, gadopentetate, gadobenate, gadoxetic acid, gadodiamide, gadoversetamide, gadofosveset, iopamidol, iohexol, ioxilan, iopromide, iodixanol, ioxaglate, metrizoate, and diatrizoate.

As an example, the second compound or the composition containing the second compound may comprise a therapeutic radioisotope. In this case, examples of therapeutic radioisotope include, but are not limited to, ⁴⁷Sc, ⁶⁷Cu, ⁹⁰Y, ¹³¹I, ¹⁵³Sm, ¹⁶¹Tb, ¹⁶⁶Ho, ⁹⁹Mo ¹⁷⁷Lu, ¹⁸⁸Re, ²¹¹At, ²¹²Pb, ²¹³Bi, ²²⁵Ac, and ²²⁷Th.

### Use for Diagnosis of FAP-Mediated Disease

According to one embodiment of the present invention, the present invention relates to a pharmaceutical composition for diagnosing FAP-mediated disease, comprising the compound provided in the present invention as an active ingredient.

According to another embodiment of the present invention, the present invention relates to a method for diagnosing FAP-mediated disease, comprising: a step of administering the above-described pharmaceutical composition to a subject; and a step of imaging.

When the pharmaceutical composition containing the compound according to the present invention is administered to a subject for the purpose of detecting FAP-expressing tissue or diagnosing FAP-mediated disease as described above, the drug (payload) that is linked to the FAP ligand of the compound may be a diagnostic radioisotope, or a chelating agent comprising the same, a fluorescent moiety, a photoacoustic reporting molecule, a Raman-active reporter molecule, a contrast agent, a detectable nanoparticle, or an enzyme.

In the present invention, the diagnostic radioisotope may particularly include, but is not limited to, ¹⁸F, ¹²³I, ¹²⁴I, ¹²⁵I or ^{99m}Tc.

The technique for imaging may be, but is not limited to, single photon emission computed tomography (SPECT), positron emission tomography (PET), or a combination of positron emission tomography and computed tomography (PET-CT).

The imaging may be performed on the entire body of the subject, but may be performed on the region of interest. The region of interest may be an organ suspected of having FAP-mediated disease, such as a liver, lungs, heart, vascular system, articular or interstitial tissue, pancreas, skin, mouth, digestive tract, brain, breast, bone marrow, peritoneum, or kidney, without being limited thereto.

In the present invention, by detecting the organs in which FAP is expressed and the degree of FAP expression, it is possible to diagnose whether FAP-mediated disease has developed or to monitor the degree of disease progression or the prognosis of the disease after treatment.

The method for diagnosing according to the present invention may further comprise: a step of obtaining anatomical images of the subject using magnetic resonance imaging or computed tomography; and a step of overlaying the obtained images to position an image related to the FAP-expressing tissue of the subject within the anatomical images.

The method of the present invention may further comprise a step of administering an agent for preventing, alleviating or treating the FAP-mediated disease to the subject when it is predicted through the imaging that FAP expression-related disease has developed or is highly likely to develop. In this case, the agent for preventing, alleviating or treating the FAP-mediated disease may be an anti-inflammatory agent, an antifibrotic agent, or a therapeutic agent with anticancer activity, and may comprise a therapeutic radioisotope. In addition, the agent may be a chemotherapeutic agent (e.g., methotrexate, cisplatin, and paclitaxel), an anti-oncogenic agent, an anti-angiogenic agent, a tumor suppressor agent, an antimicrobial agent, or an expression construct comprising a nucleic acid encoding a therapeutic protein, without being limited thereto.

### Use for Treatment of FAP-Related Disease

According to one embodiment of the present invention, the present invention relates to a pharmaceutical composition for preventing, alleviating or treating FAP-mediated disease, comprising the compound provided in the present invention as an active ingredient.

According to another embodiment of the present invention, the present invention relates to a method for preventing, alleviating or treating FAP-mediated disease, comprising a step of administering the above-described pharmaceutical composition to a subject.

In the present invention, the FAP-mediated disease may be a disease associated with proliferation, tissue remodeling, chronic inflammation, obesity, glucose intolerance, or insulin insensitivity.

In the present invention, the FAP-mediated disease may be a tumor or cancer, and more specifically, may be breast cancer, colorectal cancer, ovarian cancer, prostate cancer, pancreatic cancer, kidney cancer, lung cancer, melanoma, fibrosarcoma, osteosarcoma, connective tissue sarcoma, renal cell carcinoma, giant cell carcinoma, squamous cell carcinoma, leukemia, skin cancer, soft tissue cancer, liver cancer, gastrointestinal carcinoma, or adenocarcinoma, without being limited thereto.

In the present invention, the FAP-mediated disease may be, but is not limited to, fibrotic disease, wound healing, keloid formation, osteoarthritis, rheumatoid arthritis and related disorders involving cartilage degradation, atherosclerotic disease, Crohn's disease, or type II diabetes.

In the present invention, the fibrotic disease may be, but is not limited to, scleroderma, atherosclerosis, cardiac fibrosis, organ transplant fibrosis, muscular fibrosis, pancreatic fibrosis, myelofibrosis, hepatic fibrosis, splenic fibrosis, pulmonary fibrosis, idiopathic pulmonary fibrosis, idiopathic interstitial fibrosis, diffuse interstitial fibrosis, interstitial lung disease, chronic interstitial lung disease, pneumoconiosis, silicosis, interstitial fibrosis, sarcoidosis, mediastinal fibrosis, cardiac fibrosis, atrial fibrosis, endocardial fibrosis, renal fibrosis, macular degeneration, keloid lesions, hypertrophic scars, nephrogenic systemic fibrosis, injection fibrosis, fibrotic complications of surgery, fibrotic chronic allograft vasculopathy, ischemic reperfusion injury-associated fibrosis, arthrofibrosis, Dupuytren's disease, fibrous proliferative lesions of the oral cavity, fibrotic intestinal stenosis, glial scar formation, leptomeningeal fibrosis, fibrosis due to radiation exposure, fibrosis due to mammary cystic rupture, myelofibrosis, retroperitoneal fibrosis, or progressive massive fibrosis.

In the present invention, when the pharmaceutical composition containing the compound according to the present invention is administered to the subject for the purpose of preventing, alleviating or treating the FAP-mediated disease, preferably fibrotic disease, a drug (payload) that is linked to the FAP ligand of the compound may be an antifibrotic agent, without being limited thereto.

In the present invention, when the pharmaceutical composition containing the compound according to the present invention is administered to the subject for the purpose of preventing, alleviating or treating the FAP-mediated disease, preferably inflammatory disease, a drug (payload) that is linked to the FAP ligand of the compound may be an anti-inflammatory agent or an immunosuppressive agent, without being limited thereto.

In the present invention, when the pharmaceutical composition containing the compound according to the present invention is administered to the subject for the purpose of preventing, alleviating or treating the FAP-mediated disease, preferably a tumor or cancer, a drug (payload) that is linked to the FAP ligand of the compound may be a therapeutic radioisotope or a cytotoxic agent, without being limited thereto.

The therapeutic radioisotope may comprise, for example, ²²⁵Ac, ⁶⁸Ga, ¹⁷⁷Lu, ⁶⁴Cu, ⁶⁷Cu, ¹³¹I, ³²P, ⁹⁰Sr, ⁹⁰Y, ⁹⁹Mo, ¹⁸⁶Re, ¹⁸⁸Re, or ¹⁸⁹Re, without being limited thereto.

As used herein, the term "treatment" or "alleviation" may include, without limitation, any action of alleviating or beneficially changing a disease using the composition of the present invention.

As used herein, the term "prevention" may include, without limitation, any action of blocking, suppressing, or delaying the symptoms of a disease using the composition of the present invention.

In the present invention, the above-described pharmaceutical composition or treatment method may be used in conjunction with other therapeutic treatment modalities, including surgery, cryosurgery, radiation, thermotherapy, hormone treatment, chemotherapy, immunotherapy, vaccines, and any combination thereof.

In addition, the above-described pharmaceutical composition may further comprise any therapeutic agent (e.g., molecule, drug, pharmaceutical composition, etc.) capable of preventing, inhibiting, or arresting the symptoms and/or progression of a disease. The therapeutic agent is selected from, but not limited to, a chemotherapeutic agent (e.g., methotrexate, cisplatin and paclitaxel), an anti-oncogenic agent, an anti-angiogenic agent, a tumor suppressor agent, an antimicrobial agent, or an expression construct comprising a nucleic acid encoding a therapeutic protein.

### Advantageous Effects

The compound provided in the present invention is capable of binding specifically to fibroblast activation protein (FAP) with high affinity. Thus, when drugs for various purposes are linked to the compound, they may be used to image tissues in which fibroblast activation protein (FAP) is highly expressed, or to treat or diagnose various diseases mediated by fibroblast activation protein (FAP).

### Brief Description of Drawings

FIG. 1 shows molecular docking simulations conducted to investigate the interaction between FAP and each FAPI derivative.
FIG. 2a is a graph showing the change in fluorescence intensity as a function of the treatment concentration of UAMC 1110 after treating FAP with a substrate and UAMC 1110 as a FAP inhibitor.
FIG. 2b is a graph showing the change in fluorescence intensity as a function of the treatment concentration of SpGP after treating FAP with a substrate and SpGP as a FAP inhibitor.
FIG. 3 shows confocal microscope images of FAP-positive U-87 MG cells (A) and FAP-negative PC3 cells (B) after treatment with Cy5.5-SpGP.
FIG. 4 is a graph showing the results of measuring the mean cell fluorescence intensity after treating PC3 cells and U-87 MG cells with Cy5.5-SpGP.
FIG. 5 shows confocal microscope images of mFAP-overexpressing HT-1080 cells treated with Cy5.5-SpGP (A) and then further treated with the FAP inhibitor OncoFAP (B).
FIG. 6 is a graph showing the results of measuring the mean cell fluorescence intensity after treating mFAP-overexpressing HT-1080 cells with Cy5.5-SpGP and further treating the cells with the FAP inhibitor OncoFAP.
FIG. 7 shows *in vivo* fluorescence images of a liver fibrosis mouse model 0 or 1 hour after injection of Cy5.5-SpGP and ex vivo fluorescence images of harvested liver (Li), lung (Lu), spleen (Sp), heart (He), and kidney (Ki).
FIG. 8 is a graph showing the results of comparing the fluorescence signal intensity in the liver after injecting Cy5.5-SpGP into a liver fibrosis mouse model and a normal mouse.
FIG. 9 is a graph showing the results of comparing the fluorescence intensity in normal organs (lung, heart, spleen, and kidney) with that in the liver after injecting Cy5.5-SpGP into a liver fibrosis mouse model.

### Best Mode

According to one embodiment of the present invention, the present invention relates to a compound selected from the group consisting of a compound represented by Formula 1 below, a pharmaceutically acceptable salt thereof, an optical isomer thereof, a solvate thereof, and a hydrate thereof:

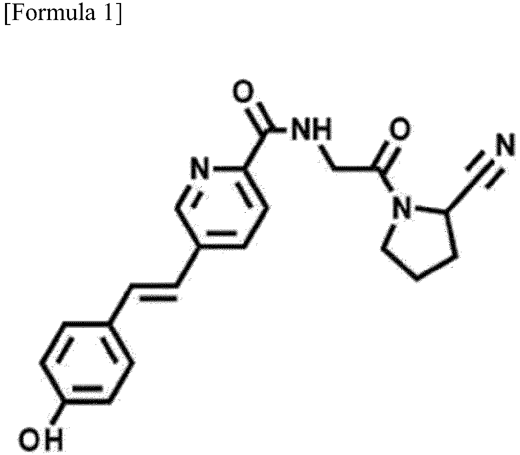

The compound represented by Formula 1 may specifically bind to fibroblast activation protein (FAP) and furthermore, inhibit the enzymatic activity of FAP.

Thus, the compound is a FAP inhibitor that may be used to prevent, alleviate, or treat FAP-related disease. In addition, various substances such as various drugs or fluorescent dyes may be linked to the compound and used to diagnose or treat FAP-related disease.

According to another embodiment of the present invention, the present invention relates to a compound selected from the group consisting of a compound represented by Formula 2 below, a pharmaceutically acceptable salt thereof, an optical isomer thereof, a solvate thereof, and a hydrate thereof:

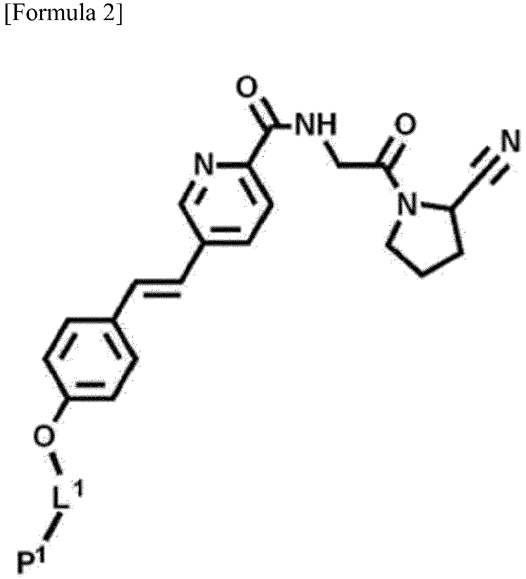

In Formula 2 above,
L¹ is a direct bond or a linker; and
P¹ is a radioactive moiety, a chelating agent, a fluorescent moiety, a photoacoustic reporting molecule, a Raman-active reporter molecule, a contrast agent, a detectable nanoparticle, an enzyme, an antifibrotic agent, an anti-inflammatory agent, an immunosuppressive agent, or a cytotoxic agent.

According to another embodiment of the present invention, the present invention relates to a pharmaceutical composition comprising, as an active ingredient, a compound selected from the above-described compound, a pharmaceutically acceptable salt thereof, an optical isomer thereof, a hydrate thereof, and a solvate thereof.

The pharmaceutical composition may be used for the prevention, treatment or diagnosis of FAP-mediated disease or disorder depending on the type of drug that is linked to a portion of the structure of the compound, which acts as a FAP ligand.

According to another embodiment of the present invention, the present invention relates to an imaging composition for FAP detection, comprising a compound provided in the present invention as an active ingredient.

### Mode for Invention

Hereinafter, the present invention will be described in more detail through examples. These examples are only intended to explain the present invention in more detail, and it will be apparent to those skilled in the art that the scope of the present invention according to the subject matter of the present invention is not limited by these examples.

### Examples

### Reagents and Instruments

All chemical solvents and reagents used in the following experiments were used without further purification and were purchased from Tokyo Chemical Industry Co., Sigma-Aldrich, and Alfa Aesar. Additionally, ¹H NMR spectra were obtained at room temperature using a Varian 400-MR Shielded 400 MHz spectrometer. Electrospray mass spectrometry (ESI-MS) was performed using an LC/MS (Agilent 1260 Infinity Series and Agilent 6130 single quadrupole LC/MS) spectrometer and an analytical column (Waters, Xterra MS C18, 3.5 µm, 2.1 × 100 mm). Fluorescence intensity was measured using a microplate reader (Bio-Tek, Synergy H1).

### Synthesis of 1-glycylpyrrolidine-2-carbonitrile

1-glycylpyrrolidine-2-carbonitrile was produced from pyrrolidine-2-carboxamide according to Reaction Scheme 1 below.

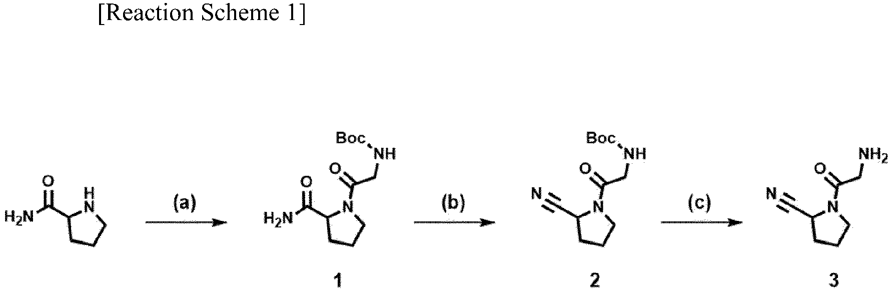

### 1. Synthesis of tert-butyl (2-(2-carbomoylpyrrolidin-1-yl)-2-oxoethyl) carbamate

Pyrrolidine-2-carboxamide (4.38 mmol, 500 mg) and benzotriazol-1-yl-oxy-tris-pyrrolidino-phosphonium hexafluorophosphate (PyBOP, 4.6 mmol) were dissolved in dichloromethane (CH₂Cl₂, 10 mL), followed by stirring at room temperature for 30 minutes. N,N-diisopropylethylamine (DIPEA, 9.2 mmol) and N-(tert-butoxycarbonyl)glycine (Boc-glycine, 4.6 mmol) were added to the mixture, followed by stirring at room temperature for 4 hours. The mixture was washed twice with 0.5 N HCl (10 mL) in water, the organic layer was separated and dried over anhydrous sodium sulfate, and the solvent was removed. The crude product was purified by silica gel column chromatography using 10% methanol in ethyl acetate as a solvent to obtain desired Compound 1 as a white solid (595 mg, 50%).

¹H NMR (400 MHz, MeOH-*d*₄) δ 5.49 (br, s, 1H), 4.76-4.75 (t, 1H), 4.37-4.33 (t, 1H), 3.93-3.82 (q, 2H), 3.7-3.66 (m, 1H), 3.65-3.50 (m, 1H), 2.27-2.15 (m, 4H), 1.45 (s, 9H).

### 2. Synthesis of tert-butyl (2-(2-cyanopyrrolidin-1-yl)-2-oxoethyl) carbamate

Compound 1 (4.94 mmol, 1.34 g) was dissolved in anhydrous tetrahydrofuran (THF) at 0°C, and then pyridine (34.57 mmol, 2.8 mL) was added and trifluoroacetic anhydride (5.68 mmol, 0.8 mL) in 70 mL of THF was added dropwise to the solution. After stirring for 90 minutes, the solvent was evaporated, and the mixture was redissolved in ethyl acetate and washed with 2 M aqueous hydrochloric acid solution (3 x 6 mL). The acidic aqueous layer was extracted twice with ethyl acetate. The organic layer was washed with a saturated aqueous solution of sodium bicarbonate and brine. The crude product was purified by silica gel column chromatography using 70% ethyl acetate in hexane as a solvent to obtain Compound 2 as a white solid (895 mg, 71.6%).

¹H NMR (400 MHz, CDCl₃) δ 5.39 (br, s, 1H), 4.75 (s 1H), 4.00-3.85 (m, 2H), 3.62-3.58 (m, 1H), 3.57-3.42 (m, 1H), 2.32-2.16 (m, 4H), 1.45 (s, 9H).

### 3. Synthesis of 1-glycylpyrrolidine-2-carbonitrile

A mixture containing Compound 2 (500 mg, 1.98 mmol) was added to 20% trifluoroacetic acid (CH₂Cl₂, 50 mL), followed by stirring at 25°C for 2 hours. After removing the solvent under reduced pressure, the residue was dissolved in a small amount of dichloromethane. When diethyl ether was added to the crude product, a precipitate was formed. This solid was washed with ethyl ether and dried under vacuum. Compound 3 was obtained as a yellow solid (242.16 mg, 80%).

¹H NMR (400 MHz, MeOH-d4) δ 4.87-4.84 (t, 1H), 4.00-3.89 (m, 2H) , 3.71-3.68 (m, 1H), 3.53-3.50 (m, 1H), 2.34-2.18 (m, 4H)

### Synthesis of 3-styrylpyridine scaffold

(E)-N-(2-(2-Cyanopyrrolidin-1-yl)-2-oxoethyl)-5-(4-hydroxystyryl)picolinamide, a 3-styrylpyridine scaffold, was synthesized according to Reaction Scheme 2 below.

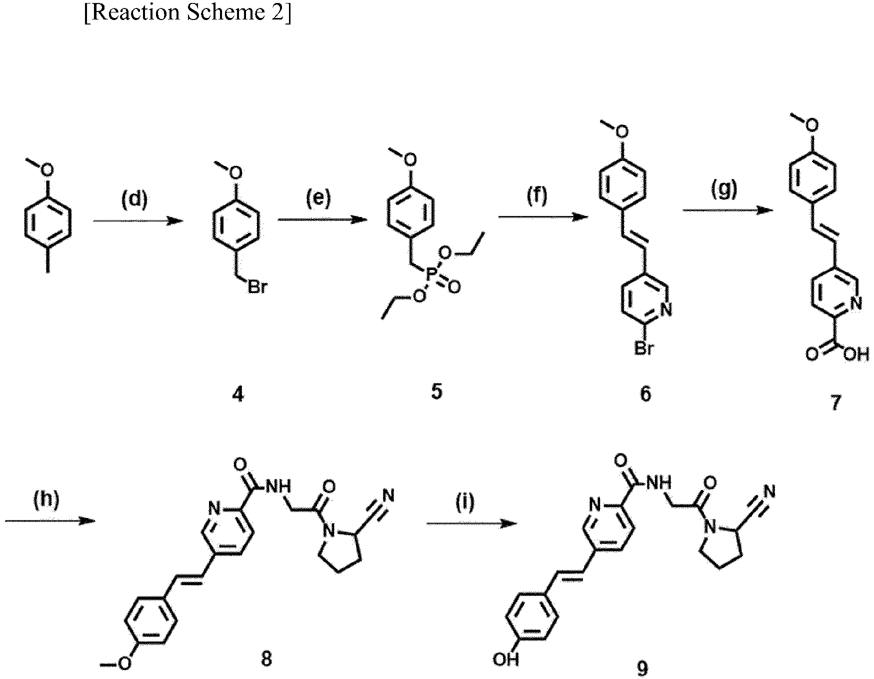

### 1. Synthesis of 1-(bromomethyl)-4-methoxybenzene

4-Methylanisole (24.56 mmol, 3.1 mL) and N-bromosuccinimide (NBS, 24.56 mmol) were added to cyclohexane, followed by stirring at 90°C for 8 hours. The resulting mixture was filtered to remove the succinimide and washed with cyclohexane. After evaporating the filtrate, the residue was purified by silica gel chromatography using 10% ethyl acetate in hexane as the solvent to obtain Compound 4 as a colorless oil (47.5% yield).

### 2. Synthesis of diethyl (4-methoxybenzyl)phosphonate

Compound 4 (25.07 mmol, 5.04 g) was placed in a pressure tube, and triethyl phosphite (25.57 mmol, 4.78 mL) was added thereto. The reaction mixture was heated slowly at 135°C for 4 hours and cooled to room temperature. The remaining triethyl phosphite was removed under vacuum. The raw material was purified by silica gel column chromatography using 30% ethyl acetate in hexane as a solvent to obtain Compound 5 as a colorless oil (5.44 g, 84% yield).

¹H NMR (400 MHz, CDCl₃) δ 7.38-7.36 (m, 0.5H), 7.22-7.20 (m, 1.5H), 6.91-6.84 (d, J = 8.6 Hz , 2H), 4.10-3.95 (m, 4H), 3.79 (s, 3H), 3.12-3.06 (d, J = 21.2Hz, 2H), 1.35-1.16 (m, 6H); MS (ESI) m/z 257.2 [M+H]⁺.

### 3. Synthesis of (E)-2-bromo-5-(4-methoxystyryl)pyridine

Anhydrous THF (25 mL), 2-bromopyridine-5-carboxaldehyde (6.97 mmol, 1.8 g) and sodium hydride (60% w/w in mineral oil, 69.7 mmol, 2.78 g) were added to a mixture containing Compound 5. After stirring the mixture at 90°C for 4 hours, a saturated aqueous solution (20 mL) of sodium bicarbonate (NaHCO3) was added thereto, followed by extraction with ethyl acetate (20 mL). The combined organic layer was dried over anhydrous sodium sulfate and the solvent was evaporated. The solid was purified by silica gel column chromatography using 2% ethyl acetate in dichloromethane as a solvent to obtain Compound 6 as a white solid (424.7 mg, 21% yield).

¹H NMR (400 MHz, CDCl₃) δ 8.43-8.42 (d, J = 4 Hz, 1H), 7.69-7.47 (m, 1H), 7.46-7.42 (m, 3H), 7.12-7.08 (d, J = 16.28 Hz, 1H), 6.93-6.90 (m, 2H), 6.89-6.85 (d, J = 16.28 Hz, 1H), 3.84 (s, 3H); MS (ESI) m/z 291.2 [M+H]⁺.

### 4. Synthesis of (E)-5-(4-Methoxystyryl)picolinic acid

Compound 6 (0.172 mmol, 50 mg) in anhydrous THF (8 mL) was cooled to - 78°C under a nitrogen gas atmosphere, and then n-butyllithium (1.6 M in n-hexane, 0.189 mmol, 0.12 mL) was added dropwise thereto. After 30 minutes, an excess of solid carbon dioxide was added, and the mixture was kept at room temperature. After 1 hour, water (0.5 mL) was added thereto, followed by extraction with ethyl acetate (0.5 mL). 1 M hydrochloric acid was added to this suspension, followed by concentration under vacuum. The resulting yellow solid was washed with ethyl acetate to obtain Compound 7 (8.3 mg, 19% yield). MS (ESI) m/z 256.2 [M+H]⁺.

### 5. Synthesis of (E)-N-(2-(2-cyanopyrrolidin-1-yl)-2-oxoethyl)-5-(4-methoxystyryl)picolinamide

A solution of Compound 7 (0.0215 mmol, 5.5 mg) in dimethylformamide (DMF, 1 mL) was treated with PyBOP (0.0287 mmol, 12.31 mg) and stirred for 30 minutes. 1-Glycylpyrrolidine-2-carbonitrile (Compound 3, 0.0215 mmol, 3.3 mg) and DIPEA (0.086 mmol, 0.015 mL) were added to the mixture, followed by stirring overnight at room temperature. The mixture was washed twice with 1 N HCl (1 mL) in water. The organic layer was separated and dried over anhydrous sodium sulfate, and then the solvent was removed. The crude product was purified by silica gel column chromatography using 80% ethyl acetate in hexane as a solvent to obtain Compound 8 as a white solid (2.4 mg, 28.6% yield).

¹H NMR (400 MHz, CDCl₃) δ 8.75 (s, 1H), 8.65 (s, 1H), 8.13-8.11 (d, J = 8 Hz, 1H), 7.94-7.92 (d, J = 8 Hz, 1H), 7.51-7.48 (m, 2H), 7.23-7.19 (d, J = 16 Hz, 1H), 7.00-6.96 (d, J = 16 Hz, 1H), 6.94-9.92 (m, 1H), 4.83-4.81 (m, 1H), 4.34-4.23 (m, 2H), 4.13-4.11 (m, 1H), 3.85 (s, 3H), 3.71-3.69 (m, 1H), 3.58-3.52 (m, 1H), 2.33-2.21 (m, 4H); MS (ESI) m/z 391.4 [M+H]⁺.

### 6. Synthesis of (E)-N-(2-(2-Cyanopyrrolidin-1-yl)-2-oxoethyl)-5-(4-hydroxystyryl)picolinamide

AlCl₃ (0.033 mmol, 4.33 mg) was added to Compound 8 (0.013 mmol, 5 mg) in CH₂Cl₂ (2 mL), followed by stirring at 80°C for 60 hours. Water was added to terminate the reaction, and the mixture was extracted with ethyl acetate. The combined organic layer was washed with a sodium bicarbonate solution and then with a saturated sodium chloride solution. The organic layer was separated and dried over anhydrous sodium sulfate, and then the solvent was removed. Compound 9 was obtained as a yellow solid (1.5 mg, 31% yield).

### In silico molecular docking simulation targeting FAP

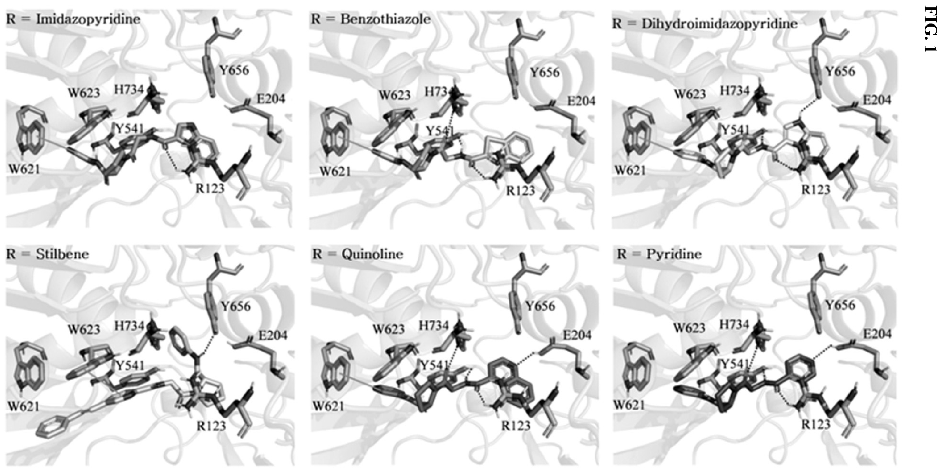

The drug structures of fibroblast activation protein inhibitors well known to date contain a quinoline moiety. Quinoline can directly block the active site of fibroblast activation protein enzyme or interfere with proteolytic activity. Accordingly, the present inventors introduced other aryl-based structures instead of quinoline to improve binding affinity for fibroblast activation protein. First, for molecules targeting FAP, R in the structure of the following formula was substituted with a total of six different aryl groups, including imidazopyridine, benzothiazole, dihydroimidazopyridine, styrylpyridine, quinoline, and pyridine, to produce FAPI derivatives. For comparison, a structure substituted with quinoline was designed. 2D models of these molecules were obtained using Chemdraw software (version 12.0), and Glide Maestro program (version 12.5) was used to evaluate the binding affinity between these molecules and the target. All the ligands were placed within the active site of the target using Glide's standard precision mode, which made it possible to find a favorable orientation in consideration of the flexibility of the ligand and obtain a positive docking score while avoiding penalties. Molecular docking simulations of aryl-based FAPI derivatives with FAP are shown in FIG. 1. In FIG. 1, bright blue lines represent π-π interactions, and dotted lines represent hydrogen bonds between atoms. Additionally, the binding free energy between each FAPI derivative and FAP was calculated, and the results are shown in Table 1 below.

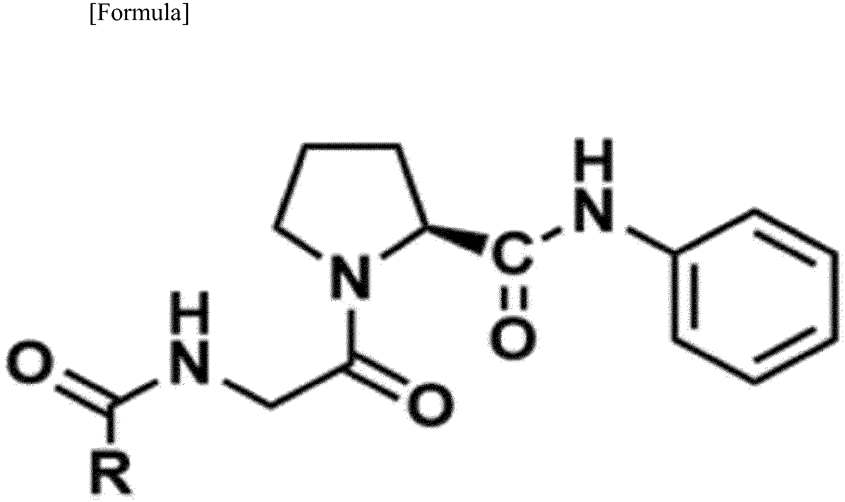

**[Table 1]**

| R group | ΔG_{bind} [kcal/mol] |
|---|---|
| Imidazopyridine | -50.38 |
| Benzothiazole | -58.23 |
| Dihydroimidazopyridine | -70.07 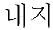 -69.11 |
| 3-styrylpyridine | -81.72 |
| Quinoline | -61.68 |
| Pyridine | -43.34 |

As shown in Table 1 above, among the six aryl-based FAPI derivatives, the derivative in which the R group is 3-styrylpyridine exhibited the lowest binding energy (-81.72 kcal/mol) and the highest binding affinity. Accordingly, the structure in which the R group is 3-styrylpyridine (hereinafter referred to as "SpGP") was selected as the FAP-targeting ligand according to the present invention, and the following experiment was conducted.

### Preparation of Cy5.5-SpGP

Based on the molecular docking simulation results described above, in order to conduct studies on the application of the SpGP structure as a FAP-targeting ligand for imaging, Cy5.5 was selected as a fluorescent dye and conjugated to SpGP (Compound 10). Cy5.5 (10 eq.) was added to Compound 10 in DMAP and allowed to react at 25°C to synthesize a Cy5.5-SpGP compound.

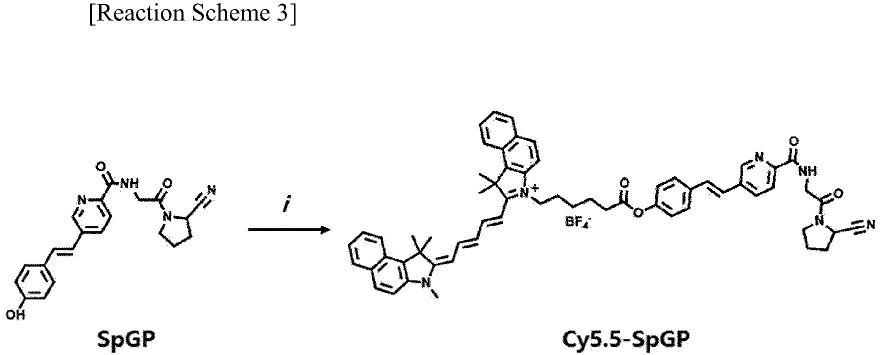

### Evaluation of in vitro FAP inhibitory activity of Cy5.5-SpGP

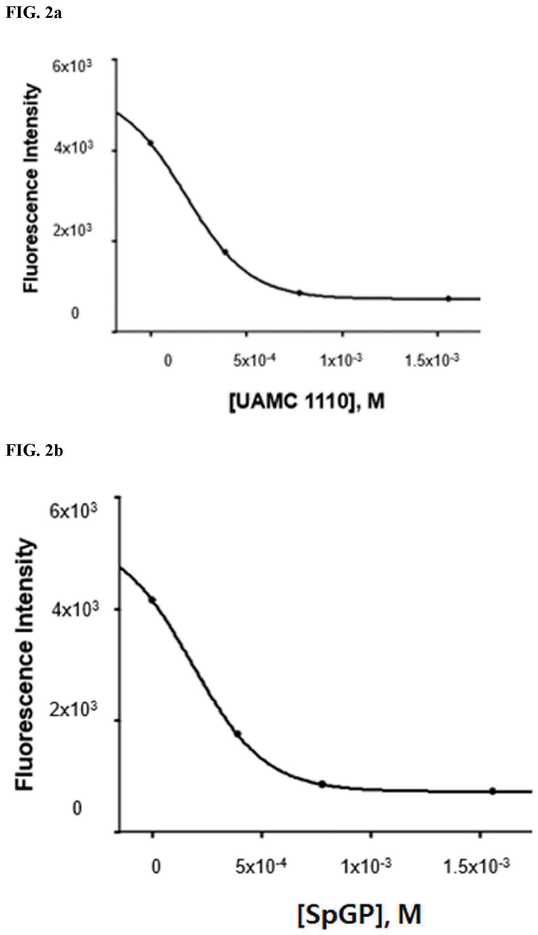

The binding affinity of Cy5.5-SpGP for FAP was measured by fluorescence intensity. When the substrate-AMC dye bond is cleaved by FAP, fluorescence is activated and fluorescence intensity increases. Conversely, as FAP inhibition increases, fluorescence intensity gradually decreases. To evaluate the enzymatic activity of FAP, FAP was reacted with Cy5.5-SpGP as an FAP inhibitor in the presence of an Ala-Pro-AMC substrate and the degree of reduction in fluorescence intensity was evaluated. The reaction mixture contained the substrate at a concentration of 2.5 µM, a fixed amount of FAP (20 nM), and an assay buffer (50 mM Tris, 100 mM NaCl, 1 mM EDTA) at pH 7.4, and serial dilutions of Cy5.5-SpGP were added from high to low concentration to make a total volume of 100 µL. Fluorescence was monitored using a microplate reader at an excitation wavelength of 350 nm and an emission wavelength of 430 nm. As a control, UAMC 1110 known as a FAP inhibitor was used. Changes in fluorescence intensity as a function of the treatment concentration of each inhibitor are shown in FIGS. 2a and 2b. The IC₅₀ of each inhibitor was calculated and the results are shown in Table 2 below.

**[Table 2]**

| Compound | IC₅₀ [nM] |
|---|---|
| UAMC 1110 | 35 nM |
| SpGP | 43 M |

As seen in FIG. 2 and Table 2 above, it could be seen that SpGP had excellent FAP inhibitory activity similar to UAMC 1110.

### In vitro imaging and evaluation of intracellular uptake of Cy5.5-SpGP

To confirm the targeting functionality of Cy5.5-SpGP to human FAP, confocal microscopy experiments were performed.

U-87 MG cells obtained from American Type Culture Collection (ATCC) HTB-14TM; ATCC-LGC Standard (Wessel, Germany) were cultured in Dulbecco's Modified Eagle Medium (DMEM, Gibco, Grand Island, NY, USA) containing 10% fetal bovine serum (FBS) and 1% penicillin-streptomycin at 37°C under 5 vol% CO₂. When confluence reached 90%, the cells were isolated using trypsin and seeded again at a 1:5 ratio. PC3 cells were seeded in RPMI medium supplemented with 10% FBS and cultured at 37°C under and 5 vol% CO₂. When confluence reached 90%, the PC3 cells were also isolated using trypsin and seeded again at a 1:5 ratio. *In vitro* experiments were performed when confluence reached 60 to 70%. FAP-positive U-87 MG cells were seeded into a 12-well plate (SPL Life Science) cover glass at a density of 5.4 x 10⁵ cells per well. The cells were cultured in DMEM medium (1 mL) supplemented with 10% FBS for 36 hours under standard culture conditions. To evaluate the intracellular uptake of SpGP, 2 mg of Cy5.5-SpGP was dissolved in 100 µL of DMSO and diluted with deionized water to a final concentration of 1 µM/mL. After removing the culture medium, the cell medium was treated with DMSO containing Cy5.5-SpGP (1 µM), incubated for 30 minutes, and then washed once with medium. The nuclei were stained using DAPI and imaged using a confocal microscope, and the intracellular uptake of Cy5.5-SpGP was evaluated by measuring the fluorescence at a wavelength of 703 nm by excitation with a 683 nm laser.

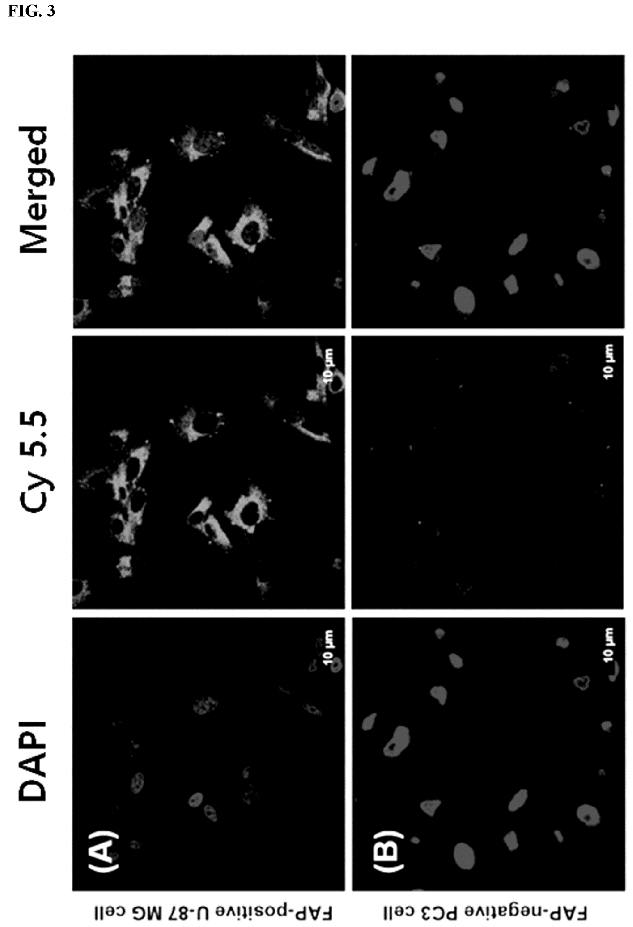

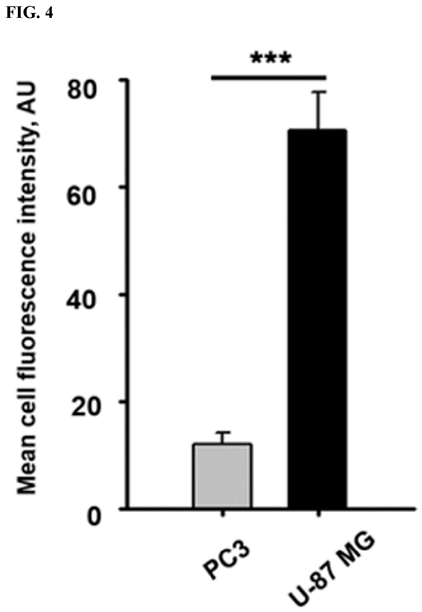

As shown in FIGS. 3 and 4, red Cy5.5-SpGP fluorescence was significantly observed in the U-87 MG cells expressing FAP. On the other hand, no significant accumulation of Cy5.5-SpGP was observed in the FAP-negative PC3 cells.

Additionally, to evaluate the FAP specificity of Cy5.5-SpGP, HT-1080 cells overexpressing mFAP were treated with 1 µM of Cy5.5-SpGP and treated with 1,000 µM of the FAP inhibitor OncoFAP. Then, to determine the extent of intracellular uptake of Cy5.5-SpGP following each treatment, the nuclei were stained with DAPI and observed using a confocal microscope.

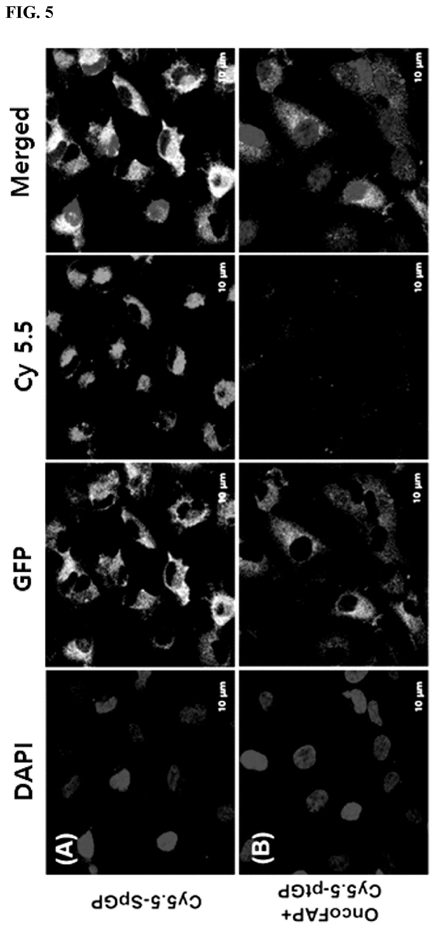

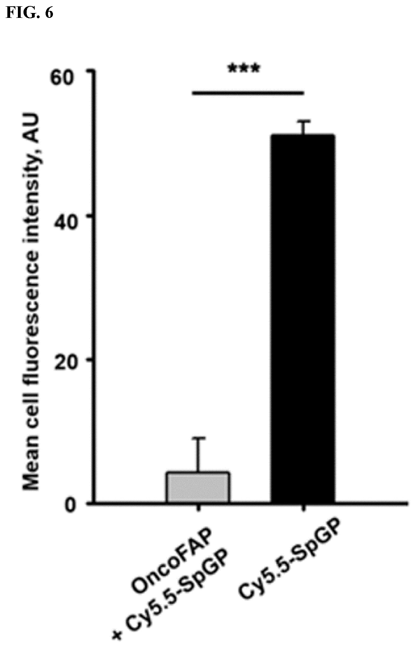

As a result, as shown in FIGS. 5 and 6, treatment with the FAP inhibitor reduced the intracellular uptake of Cy5.5-SpGP, and in particular, only DAPI and GFP were observed in the merged image. The reason why Cy5.5 fluorescence was not observed in the cytoplasm and cell membrane can be attributed to the fact that OncoFAP bound to FAP and blocked the binding of Cy5.5-SpGP to FAP.

Through these experiments, it could be seen that SpGP according to the present invention has specificity for FAP and thus selectively accumulates in FAP-expressing cells.

### Imaging for in vivo and ex vivo visualization of Cy5.5-SpGP

To evaluate the FAP-targeting effect of Cy5.5-SpGP *in vivo*, a carbon tetrachloride (CCl₄)-induced chronic liver fibrosis mouse model was established. Specifically, a 1:3 (v/v) mixture of CCl₄ and mineral oil was injected intraperitoneally into 7-week-old male BALB/c mice at a dose of 1.5 mL/kg twice a week for 3 weeks. Only mineral oil was administered to a control group. Two groups of 7-week-old mice with CCl₄-induced liver fibrosis as described above were used for *in vivo* IVIS fluorescence imaging. Cy5.5-SpGP was intravenously injected into the tail veins of liver fibrosis mice (n=2), and *in vivo* images were acquired at 0 and 1 hour post-injection using IVIS. Thereafter, the mice were sacrificed and organs to be evaluated were extracted. Five major organs, including the heart, lung, liver, spleen, and kidney, were harvested and subjected to *in vitro* imaging. Fluorescence signals were quantified by defining and measuring regions of interest in fibrotic areas and harvested organs. Living Images^{®} software (version 4.7.3) was used for quantitative analysis. Then, statistical analysis was performed to compare fluorescence levels between the fibrotic areas and the major organs.

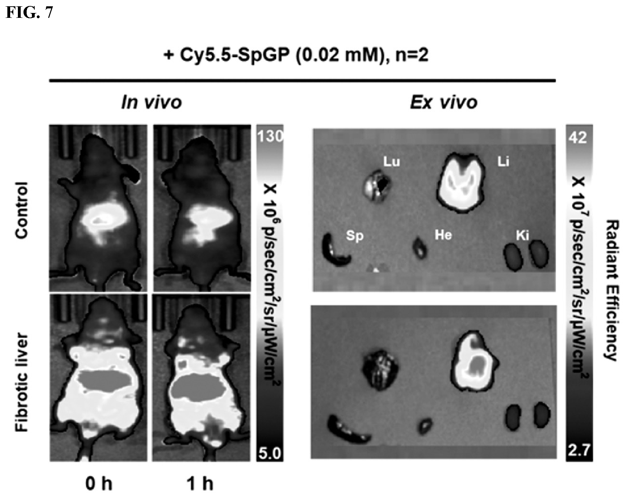

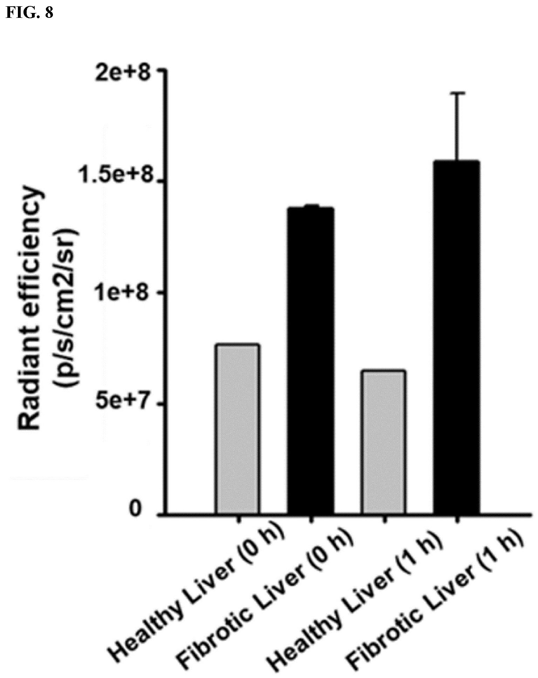

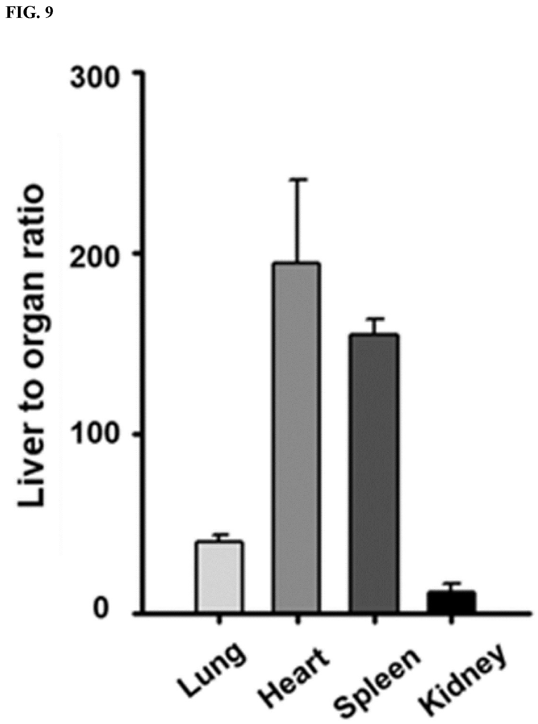

As a result, as shown in FIGS. 7 to 9, it could be confirmed that Cy5.5-SpGP was highly uptaken in the FAP-overexpressing fibrotic liver compared to the other organs. However, it was shown that Cy5.5-SpGP was rapidly eliminated in the major healthy organs within 1 hour after injection. In addition, Cy5.5 signal intensity was detected at a higher level in the liver fibrosis mouse model than in the normal control group, specifically showing a 2.4-fold higher uptake rate.

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

### Industrial Applicability

The present invention relates to a ligand compound capable of specifically binding to fibroblast activation protein (FAP). This ligand compound may be used for imaging to detect FAP, or for the prevention, alleviation, or treatment of FAP-mediated disease, or for the diagnosis of such disease.

[National Research and Development Project that supported this invention]
[Project Serial Number] 1465037025
[Project Number] HN22C0632000022
[Government Department] Ministry of Health and Welfare
[Project Management (Specialized) Agency] Korea Drug Development Foundation
[Research Project Name] National Drug Development Project (Ministry of Science and ICT, Ministry of Health and Welfare, and Ministry of Trade, Industry and Energy)
[Research Task Name] Research on Identification of Active Substances for Translocator Protein-Targeting Cancer Therapeutics
[Agency Carrying Out Project] Seoul National University R&DB Foundation
[Research Period] May 1, 2022 to April 30, 2025
[National Research and Development Project that supported this invention]
[Project Serial Number] 1415180797
[Project Number] 20018522
[Government Department] Ministry of Trade, Industry and Energy
[Project Management (Specialized) Agency] Korea Planning & Evaluation Institute of Industrial Technology
[Research Project Name] Development of Advanced Production Technology for Vaccine Raw Materials
[Research Task Name] Development of Technology for Evaluation of mRNA Vaccine Product Quality and Efficacy
[Agency Carrying Out Project] Seoul National University
[Research Period] April 1, 2022 to December 31, 2025
[National Research and Development Project that supported this invention]
[Project Serial Number] 1711168268
[Project Number] 2020R1C1C1009000
[Government Department] Ministry of Science and ICT
[Project Management (Specialized) Agency] National Research Foundation of Korea
[Research Project Name] Excellent Young Researcher Program
[Research Task Name] Development of Targeted Radionuclide-Photodynamic Combination Cancer Therapy Using Radioluminescent Liposomal Nanoplatform
[Agency Carrying Out Project] Seoul National University
[Research Period] March 1, 2020 to February 28, 2025
[National Research and Development Project that supported this invention]
[Project Serial Number] 1711158495
[Project Number] 2021M2E8A1039564
[Government Department] Ministry of Science and ICT
[Project Management (Specialized) Agency] National Research Foundation of Korea
[Research Project Name] Research on Future Innovative Foundation Technologies for Radiation Utilization
[Research Task Name] Development of Radiation-Induced Photoimmunotherapy Using Antibody-Europium-Photosensitizer Complex
[Agency Carrying Out Project] Seoul National University
[Research Period] May 1, 2021 to December 31, 2024
[National Research and Development Project that supported this invention]
[Project Serial Number] 2710009979
[Project Number] 2021R1A2C2003301
[Government Department] Ministry of Science and ICT
[Project Management (Specialized) Agency] National Research Foundation of Korea
[Research Project Name] Mid-career Researcher Support Program (R1A2)
[Research Task Name] Development of pH- and Temperature-Sensitive Nanocomposite-Based Nanocarriers That Enhance Loading Capacity, Tumor Selectivity, and Retention of Mitochondrial-Targeted Tumor Therapeutic Agents
[Agency Carrying Out Project] Seoul National University Bundang Hospital
[Research Period] March 1, 2021 to February 28, 2026
[National Research and Development Project that supported this invention]
[Project Serial Number] RS-2024-00512498
[Project Number] 0665-20240042
[Government Department] Ministry of Health and Welfare
[Project Management (Specialized) Agency] Korea Health Industry Development Institute
[Research Project Name] Korean ARPA-H Project
[Research Task Name] Development of Radioligand Therapy for Refractory Triple Negative Breast Cancer and Pancreatic Cancer through Generative AI-Based HighSpeed Drug Discovery and Rapid Advancement
[Agency Carrying Out Project] Seoul National University R&DB Foundation

## Claims

1. A compound selected from the group consisting of a compound represented by Formula 1 below, a pharmaceutically acceptable salt thereof, an optical isomer thereof, a solvate thereof, and a hydrate thereof:

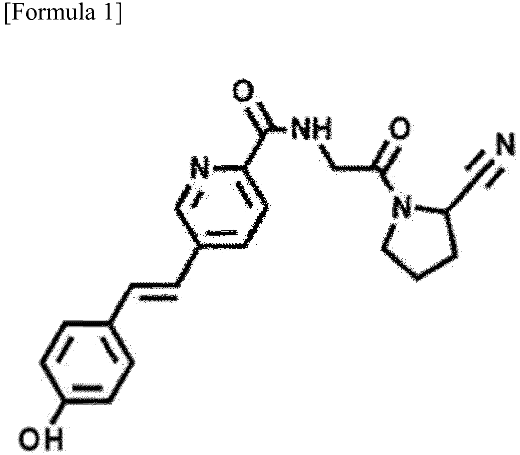

2. The compound of claim 1, wherein the compound specifically binds to fibroblast activation protein (FAP).

3. A compound selected from the group consisting of a compound represented by Formula 2 below, a pharmaceutically acceptable salt thereof, an optical isomer thereof, a solvate thereof, and a hydrate thereof: 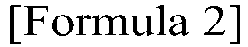 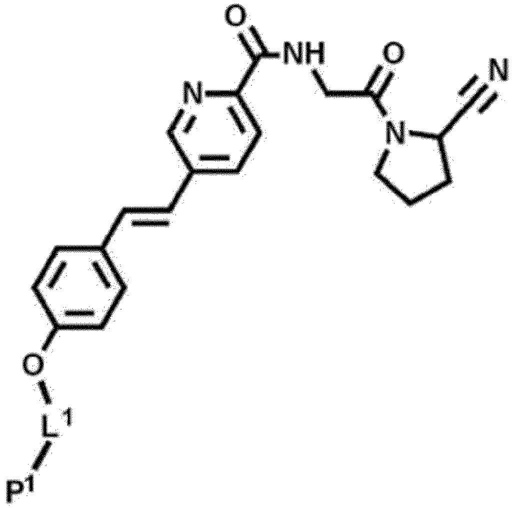 in Formula 2 above,
L¹ is a direct bond or a linker; and
P¹ is a radioactive moiety, a chelating agent, a fluorescent moiety, a photoacoustic reporting molecule, a Raman-active reporter molecule, a contrast agent, a detectable nanoparticle, an enzyme, an antifibrotic agent, an anti-inflammatory agent, an immunosuppressive agent, or a cytotoxic agent.

4. The compound of claim 3, wherein P¹ is the radioactive moiety or the fluorescent moiety.

5. The compound of claim 3, wherein the compound is a compound represented by Formula 3 below:

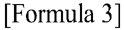

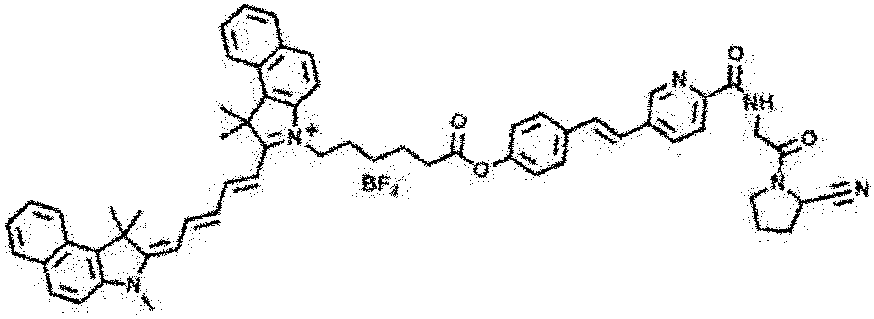

6. A pharmaceutical composition for preventing or treating fibrotic disease, comprising the compound of claim 1 or 2 as an active ingredient.

7. The pharmaceutical composition of claim 6, wherein the fibrotic disease is scleroderma, atherosclerosis, cardiac fibrosis, organ transplant fibrosis, muscular fibrosis, pancreatic fibrosis, myelofibrosis, hepatic fibrosis, splenic fibrosis, pulmonary fibrosis, idiopathic pulmonary fibrosis, idiopathic interstitial fibrosis, diffuse interstitial fibrosis, interstitial lung disease, chronic interstitial lung disease, pneumoconiosis, silicosis, interstitial fibrosis, sarcoidosis, mediastinal fibrosis, cardiac fibrosis, atrial fibrosis, endocardial fibrosis, renal fibrosis, macular degeneration, keloid lesions, hypertrophic scars, nephrogenic systemic fibrosis, injection fibrosis, fibrotic complications of surgery, fibrotic chronic allograft vasculopathy, ischemic reperfusion injury-associated fibrosis, arthrofibrosis, Dupuytren's disease, fibrous proliferative lesions of the oral cavity, fibrotic intestinal stenosis, glial scar formation, leptomeningeal fibrosis, fibrosis due to radiation exposure, fibrosis due to mammary cystic rupture, myelofibrosis, retroperitoneal fibrosis, or progressive massive fibrosis.

8. A composition for imaging comprising the compound of any one of claims 3 to 5 as an active ingredient.

9. A pharmaceutical composition for diagnosing fibrotic disease, comprising the compound of any one of claims 3 to 5 as an active ingredient.

10. The pharmaceutical composition of claim 9, wherein the fibrotic disease is scleroderma, atherosclerosis, cardiac fibrosis, organ transplant fibrosis, muscular fibrosis, pancreatic fibrosis, myelofibrosis, hepatic fibrosis, splenic fibrosis, pulmonary fibrosis, idiopathic pulmonary fibrosis, idiopathic interstitial fibrosis, diffuse interstitial fibrosis, interstitial lung disease, chronic interstitial lung disease, pneumoconiosis, silicosis, interstitial fibrosis, sarcoidosis, mediastinal fibrosis, cardiac fibrosis, atrial fibrosis, endocardial fibrosis, renal fibrosis, macular degeneration, keloid lesions, hypertrophic scars, nephrogenic systemic fibrosis, injection fibrosis, fibrotic complications of surgery, fibrotic chronic allograft vasculopathy, ischemic reperfusion injury-associated fibrosis, arthrofibrosis, Dupuytren's disease, fibrous proliferative lesions of the oral cavity, fibrotic intestinal stenosis, glial scar formation, leptomeningeal fibrosis, fibrosis due to radiation exposure, fibrosis due to mammary cystic rupture, myelofibrosis, retroperitoneal fibrosis, or progressive massive fibrosis.

11. A method for imaging FAP-expressing cells, organ, or tissue, comprising: a step of administering the composition of claim 8 to a subject; and a step of imaging a tissue of the subject.

12. A method for diagnosing FAP-mediated disease, comprising: a step of administering the pharmaceutical composition of claim 9 to a subject; and a step of imaging.

13. A method for preventing or treating FAP-mediated disease, comprising a step of administering the pharmaceutical composition of claim 6 to a subject.
